# EUROPEAN PATENT APPLICATION

(11) **EP 3 669 866 A1**
(43) Date of publication of application: **24.06.2020**
(21) Application number: 18214161.4
(22) Date of filing: 19.12.2018
(51) Int. Cl.: A61K 9/20, A61K 9/28, A61K 47/32, A61K 47/38, A61K 31/437, A61P 7/02

(54) **PHARMACEUTICAL COMPOSITION COMPRISING APIXABAN**

(71) Applicant: KRKA, d.d., Novo mesto, 8501 Novo mesto (SI)
(72) Inventor: Lorbek, Gregor, 8000 Novo mesto (SI); Videc, Danijel, 1000 Ljubljana (SI); Klemen, Korasa, 1000 Ljubljana (SI); Meznar, Klavdija, 8000 Novo mesto (SI); Celic, Tadeja Birsa, 5263 Dobravlje (SI)
(74) Representative: Hoefer & Partner Patentanwälte mbB

(57) **Abstract**

The present invention relates to pharmaceutical compositions comprising apixaban, and to methods for preparing these pharmaceutical compositions. Furthermore, the present invention pertains to the use of pharmaceutical compositions comprising apixaban as a medicament.

## Description

### Field of the invention

The present invention relates to pharmaceutical compositions comprising apixaban, and to methods for preparing these pharmaceutical compositions. Furthermore, the present invention pertains to the use of pharmaceutical compositions comprising apixaban as a medicament.

### Background of the invention

Apixaban is a compound having the chemical name 1-(4-methoxyphenyl)-7-oxo-6-[4-(2-oxo-1-piperidinyl)phenyl]-4,5,6,7-tetrahydro-1H-pyrazolo[3,4-c]pyridine-3-carboxamide, and has the structure shown below in Formula (I):

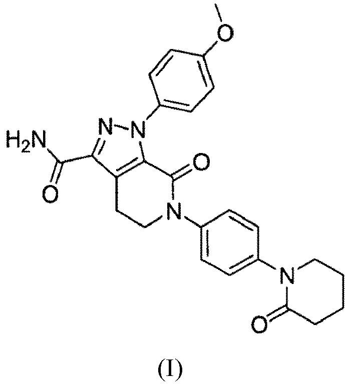

Apixaban is a factor Xa inhibitor and can be used for the treatment or prevention of a thromboembolic disorder. Apixaban is disclosed in U.S. Patent No. 6,967,208. Apixaban has been described as a poorly soluble compound. Due to this poor solubility of apixaban, a need in the art exists for the provision of a pharmaceutical composition comprising apixaban that provides an appropriate dissolution rate of apixaban.

Pharmaceutical compositions comprising the poorly soluble active ingredient apixaban are known in the art. In particular, several publications teach preparing pharmaceutical compositions comprising apixaban having a particular particle size:
For example, WO 2011/106478 A2 discloses compositions comprising crystalline apixaban particles having a mean particle size equal to or less than about 89 µm, and a pharmaceutically acceptable diluent or carrier.

WO 2015/097090 A1 mentions solid particles of poorly soluble drugs having an average particle size of 100 µm or less, wherein a solubilizer is adsorbed on the surface of the poorly soluble drug.

WO 2017/182908 A1 describes compositions prepared by using apixaban of particles d(0.9) about 20 µm.

Furthermore, several publications address the problem of low solubility of apixaban by preparing solid dispersions or adsorbates on carriers, such as for example US 2015/0018386, and WO 2014/203275.

Further approaches for preparing pharmaceutical compositions comprising apixaban are disclosed in WO 2015/121472 A1, WO 2017/163170 A1, EP 3 243 505 A1, and WO 2017/221209 A1, WO2015/103230, Research Disclosure database number 605034 (www.researchdisclosure.com), WO2017/088841, and WO2017/125535.

Although various approaches for preparing pharmaceutical compositions comprising apixaban are known in the art, there still remains a need in the art for a pharmaceutical composition comprising apixaban which provides advantageous, especially improved dissolution properties, as well as provides an advantageous, especially an improved bioavailability for the active ingredient apixaban. Furthermore, this pharmaceutical composition should apply readily available excipients and/or should allow large scale production. Furthermore, the pharmaceutical composition should show an appropriate storage stability and/or should show a low tendency for degradation of the active ingredient, e.g. upon contact with moisture, and/or oxygen containing air.

### Summary of the invention

The aspects, advantageous features and preferred embodiments of the present invention summarized in the following items, respectively alone or in combination, further contribute to solving the problem of the present invention:
1. Pharmaceutical composition comprising apixaban,
   said pharmaceutical composition comprising or consisting of one or more apixaban-containing granules,
   wherein each of said apixaban-containing granules comprises or consists of
   (i) apixaban, and
   (ii) hydroxyethyl cellulose.
2. Pharmaceutical composition according to item 1, wherein each of said apixaban-containing granules has a weight ratio of apixaban : hydroxyethyl cellulose which is in the range from 1:10 to 10:1, preferably in the range from 1:5 to 5:1, more preferably in the range from 1:2 to 2:1, especially in the range from 1:1.6 to 1.6:1.
3. Pharmaceutical composition according to any one of the preceding items, wherein each of said apixaban-containing granules is prepared by wet granulation in the presence of a granulation fluid comprising or consisting of water.
4. Pharmaceutical composition according to any one of the preceding items, wherein each of said apixaban-containing granules comprises or consists of
   a) apixaban, hydroxyethyl cellulose, and a diluent, or
   b) apixaban, hydroxyethyl cellulose, and a disintegrant, or
   c) apixaban, hydroxyethyl cellulose, and a surfactant, or
   d) apixaban, hydroxyethyl cellulose, a diluent, and a disintegrant, or
   e) apixaban, hydroxyethyl cellulose, a diluent, and a surfactant.
5. Pharmaceutical composition according to any one of the preceding items, wherein each of said apixaban-containing granules comprises or consists of apixaban, hydroxyethyl cellulose, a diluent, a disintegrant and a surfactant.
6. Pharmaceutical composition according to any one of the preceding items, wherein each of said apixaban-containing granules comprises or consists of apixaban, hydroxyethyl cellulose, and a diluent and has a weight ratio of apixaban : diluent which is in the range from 1:10 to 1:60, preferably from 1:20 to 1:50, more preferably from 1:30 to 1:40, especially from 1:34 to 1:38.
7. Pharmaceutical composition according to any one of items 4 to 6, wherein the diluent is or comprises diluent selected from the group consisting of carbohydrates and derivatives thereof, sugar alcohols, celluloses, starch and derivatives thereof, magnesium aluminometasilicate, calcium salts of phosphoric acid, alkali carbonates, earth alkali carbonates, alkali hydrogencarbonates, earth alkali hydrogencarbonates, and mixtures thereof,
   optionally the diluent is or comprises diluent selected from the group consisting of lactose, cellulose, and mixtures thereof,
   optionally wherein the diluent is a mixture comprising or consisting of lactose monohydrate and microcrystalline cellulose.
8. Pharmaceutical composition according to any one of the preceding items, wherein the pharmaceutical composition comprises:
   (A) one or more apixaban-containing granules, wherein each of said apixaban-containing granules comprises or consists of
      (i) apixaban, and
      (ii) hydroxyethyl cellulose, and
   (B) one or more extragranular excipients, wherein preferably said one or more extragranular excipients comprise a disintegrant.
9. Pharmaceutical composition according to any one of the preceding items, wherein said pharmaceutical composition is a comprimate, especially a tablet.
10. Pharmaceutical composition according to any one of the preceding items, wherein each of said apixaban-containing granules comprise:
   apixaban in an amount of 0.7-30wt.%, preferably 1-15wt.%, more preferably 1.5-9wt.%, even more preferably 2-5wt.%, based on the total weight of the one or more apixaban-containing granules,
      hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
   optionally diluent in an amount of 50-99wt.%, preferably 70-97wt.%, more preferably 85-95wt.%, based on the total weight of the one or more apixaban-containing granules, optionally disintegrant in an amount of 0.5-10wt.%, preferably 1-8wt.%, more preferably 1.5-6wt.%, even more preferably 2-4wt.%, based on the total weight of the one or more apixaban-containing granules,
      optionally surfactant in an amount of 0.1-5wt.%, preferably 0.5-2wt.%, more preferably 0.7-1.8wt.%, based on the total weight of the one or more apixaban-containing granules, and
      optionally one or more further excipients.
11. Pharmaceutical composition according to any one of the preceding items, wherein each of said apixaban-containing granules comprises or consists of:
   - apixaban in an amount of 0.7-30wt.%, preferably 1-15wt.%, more preferably 1.5-9wt.%, even more preferably 2-5wt.%, based on the total weight of the one or more apixaban-containing granules,
   - hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
   - lactose, in particular lactose monohydrate, in an amount of 25-60wt.%, preferably 38-55wt.%, more preferably 44-51wt.%, based on the total weight of the one or more apixaban-containing granules,
   - microcrystalline cellulose in an amount of 25-55wt.%, preferably 38-49wt.%, more preferably 40-48wt.%, based on the total weight of the one or more apixaban-containing granules,
   - sodium croscarmellose in an amount of 0.5-10wt.%, preferably 1-8wt.%, more preferably 1.5-6wt.%, even more preferably 2-4wt.%, based on the total weight of the one or more apixaban-containing granules,
   - sodium lauryl sulfate in an amount of 0.1-5wt.%, preferably 0.5-2wt.%, more preferably 0.7-1.8wt.%, based on the total weight of the one or more apixaban-containing granules, and
   - optionally one or more further excipients.
12. Pharmaceutical composition according to any one of items 8-11, wherein the pharmaceutical composition comprises:
   one or more apixaban-containing granules in an amount of 30-99wt.%, preferably in an amount of 70-98wt%, more preferably in an amount of 85-97wt.%, especially in an amount of 90-95wt.%, based on the total weight of the pharmaceutical composition, and
   one or more extragranular excipients, the total weight of extragranular excipients being in an amount of 1-70wt%, preferably in an amount of 2-30wt.%, more preferably in an amount of 3-15wt.%, especially in an amount of 5-10wt.%, based on the total weight of the pharmaceutical composition.
13. Process for preparing a pharmaceutical composition comprising apixaban, in particular pharmaceutical composition for oral administration, especially process for preparing a pharmaceutical composition comprising apixaban, said pharmaceutical composition comprising or consisting of one or more apixaban-containing granules, said process comprising
   (i) preparing a granulate comprising apixaban and hydroxyethyl cellulose, preferably by wet granulation;
   (ii) optionally drying the granulate obtained from step (i),
   (iii) optionally sieving and/or milling the granulate obtained from step (i) or the optionally dried granules obtained from step (ii),
   (iv) optionally combining the granulate obtained from step (i) or the optionally dried granules obtained from step (ii) or the optionally sieved and/or milled granules obtained from optional step (iii) with at least one excipient to obtain a compression mixture;
   (v) optionally compressing the compression mixture to obtain a comprimate, in particular tablet; and
   (vi) optionally applying a coating on the comprimate.
14. Process for preparing a pharmaceutical composition comprising apixaban according to item 13, wherein step i) is
   i) preparing a granulate comprising apixaban and hydroxyethyl cellulose by subjecting a mixture comprising apixaban and hydroxyethyl cellulose to wet granulation in the presence of a granulation fluid comprising a solvent,
      wherein the solvent is or comprises water.
15. Process for preparing a pharmaceutical composition comprising apixaban according to any one of items 13 to 14, wherein step (i) of preparing a granulate comprising apixaban comprises:
   (I) preparing a mixture comprising apixaban, hydroxyethyl cellulose and optionally one or more further excipients;
   (II) granulating the mixture obtained from step (I) in the presence of a granulation solution comprising a solvent, said solvent preferably being or comprising water.
16. Process for preparing a pharmaceutical composition comprising apixaban according to any one of items 13 to 15, wherein step (i) of preparing a granulate comprising apixaban is step (i) of preparing a granulate comprising or consisting of
   a) apixaban, hydroxyethyl cellulose, and a diluent, or
   b) apixaban, hydroxyethyl cellulose, and a disintegrant, or
   c) apixaban, hydroxyethyl cellulose, and a surfactant, or
   d) apixaban, hydroxyethyl cellulose, a diluent, and a disintegrant, or
   e) apixaban, hydroxyethyl cellulose, a diluent, and a surfactant, or
   f) apixaban, hydroxyethyl cellulose, a diluent, a disintegrant and a surfactant,
   preferably by wet granulation.
17. Pharmaceutical composition according to any one of items 1 to 12 for use in treating or preventing a thromboembolic disorder or disease, in particular a venous thromboembolic disorder or disease.
18. Pharmaceutical composition comprising apixaban, said pharmaceutical composition comprising or consisting of apixaban-containing extrudate, in particular apixaban-containing hot-melt extrudate,
   wherein said apixaban-containing extrudate, in particular apixaban-containing hot-melt extrudate, comprises or consists of
   (I.) apixaban, and
   (II.) a polymer component which is or comprises polymer selected from polyvinyl caprolactam-polyvinyl acetate-polyethylene glycol graft copolymer, copovidone, poly(vinyl alcohol), and mixtures thereof, and
   wherein said apixaban-containing extrudate, in particular apixaban-containing hot-melt extrudate, has a weight ratio between apixaban and polymer component (apixaban : polymer component), which weight ratio is 1:4 or less, especially is 1:6 or less, especially is in the range from 1:6 to 1:9,
   wherein optionally said apixaban-containing extrudate, in particular apixaban-containing hot-melt extrudate, is obtainable from a process comprising extrusion, in particular hot melt extrusion, carried out at a temperature of at least 190°C, especially at a temperature in the range from 190°C to 230°C.
19. Pharmaceutical composition comprising apixaban according to item 18, wherein said apixaban-containing extrudate is obtainable from a process comprising hot melt extrusion carried out at a temperature in the range from 190°C to 230°C.
20. Pharmaceutical composition comprising apixaban according to any one of the preceding items 18-19, wherein said polymer component is or comprises poly(vinyl alcohol).
21. Pharmaceutical composition comprising apixaban according to any one of the preceding items 18-19, wherein said polymer component is or comprises polyvinyl caprolactam-polyvinyl acetate-polyethylene glycol graft copolymer.
22. Pharmaceutical composition comprising apixaban according to any one of the preceding items 18-19, wherein said polymer component is or comprises copovidone.
23. Pharmaceutical composition comprising apixaban according to any one of the preceding items 18-22, wherein the weight ratio between apixaban and said polymer component (apixaban : polymer component) is 1:6 or less, preferably 1:9 or less, especially in the range from 1:6 to 1:11, further especially in the range from 1:6 to 1:9.
24. Pharmaceutical composition comprising apixaban according to any one of the preceding items 19-23, wherein hot melt extrusion is carried out at a temperature of at least 190°C, or wherein hot melt extrusion is carried out at a temperature of at least 190°C, and the weight ratio apixaban : polymer component is 1:9 or less, especially in the range from 1:9 to 1:11.
25. Pharmaceutical composition comprising apixaban according to any one of the preceding items 19-23, wherein hot melt extrusion is carried out at a temperature of at least 210°C, and the weight ratio apixaban : polymer component is 1:6 or less, preferably 1:9 or less, especially in the range from 1:6 to 1:11, further especially in the range from 1:6 to 1:9.
26. Pharmaceutical composition according to any one of items 18-25, wherein said extrudate comprises amorphous form of apixaban, optionally a mixture comprising amorphous form of apixaban and apixaban form N-1.
27. Pharmaceutical composition according to any one of the preceding items 18-26, wherein said apixaban-containing extrudate, in particular said apixaban-containing hot melt extrudate is in the form of granules.
28. Pharmaceutical composition comprising apixaban, especially pharmaceutical composition according to any one of the preceding items 18-27,
   said pharmaceutical composition comprising or consisting of one or more apixaban-containing granules,
   wherein each of said apixaban-containing granules comprises or consists of
   (α) apixaban-containing extrudate, in particular apixaban-containing hot-melt extrudate, comprising or consisting of
      (I.) apixaban, and
      (II.) a polymer component which is or comprises polymer selected from polyvinyl caprolactam-polyvinyl acetate-polyethylene glycol graft copolymer, copovidone, poly(vinyl alcohol), and mixtures thereof, wherein optionally said apixaban-containing extrudate, in particular apixaban-containing hot-melt extrudate, optionally has a weight ratio between apixaban and polymer component (apixaban : polymer component), which weight ratio is 1:4 or less, especially is 1:6 or less, especially is in the range from 1:6 to 1:9, and
   (β) optionally one or more intragranular excipients.
29. Pharmaceutical composition, especially pharmaceutical composition according to any one of the preceding items 18-28, comprising:
   (A') one or more apixaban-containing granules, wherein each of said apixaban-containing granules comprises or consists of
      (α)apixaban-containing extrudate, in particular apixaban-containing hot-melt extrudate, comprising or consisting of
         (I.) apixaban, and
         (II.) a polymer component which is or comprises polymer selected from polyvinyl caprolactam-polyvinyl acetate-polyethylene glycol graft copolymer, copovidone, poly(vinyl alcohol), and mixtures thereof, and
      (β) optionally one or more intragranular excipients, and
   (B) one or more extragranular excipients, wherein preferably said one or more extragranular excipients comprise a disintegrant.
30. Pharmaceutical composition according to any one of the preceding items 18-29, wherein said pharmaceutical composition is a comprimate, especially a tablet.
31. Pharmaceutical composition according to any one of items 18-30, wherein the pharmaceutical composition comprises:
   one or more apixaban-containing granules in an amount of 30-99wt.%, preferably in an amount of 70-98wt%, more preferably in an amount of 85-97wt.%, especially in an amount of 90-97wt.%, based on the total weight of the pharmaceutical composition, and
   one or more extragranular excipients, the total weight of extragranular excipients being in an amount of 1-70wt%, preferably in an amount of 2-30wt.%, more preferably in an amount of 3-15wt.%, especially in an amount of 3-10wt.%, based on the total weight of the pharmaceutical composition.
32. Pharmaceutical composition according to any one of the preceding items for use in treating or preventing a thromboembolic disorder or disease, in particular a venous thromboembolic disorder or disease.
33. Process for the preparation of an apixaban-containing extrudate, and optionally for the preparation of a granulate or a comprimate, said apixaban-containing extrudate comprising or consisting of (I) apixaban, and (II) a polymer component which is or comprises polymer selected from polyvinyl caprolactam-polyvinyl acetate-polyethylene glycol graft copolymer, copovidone, poly(vinyl alcohol), and mixtures thereof,
   which process comprises:
   (A) preparing a mixture comprising (I) apixaban, and (II) a polymer component which is or comprises polymer selected from polyvinyl caprolactam-polyvinyl acetate-polyethylene glycol graft copolymer, copovidone, poly(vinyl alcohol), and mixtures thereof, optionally apixaban and said polymer component being present in said mixture in a weight ratio apixaban : polymer component which is 1:4 or less, especially is 1:6 or less, especially is in the range from 1:6 to 1:9;
   (B) subjecting the mixture obtained from step (A) to extrusion, especially hot melt extrusion, optionally the hot melt extrusion being carried out at a temperature of at least 190°C, further optionally at a temperature in the range of from 190°C to 230°C;
   (C) optionally comminuting the apixaban-containing extrudate obtained in step (B) to obtain comminuted apixaban-containing extrudate;
   (D) optionally converting the apixaban-containing extrudate obtained in step (B) or the comminuted apixaban-containing extrudate obtained in step (C) to form a granulate;
   (E) optionally mixing the apixaban-containing extrudate obtained in step (B) or the comminuted apixaban-containing extrudate obtained in step (C) or the granulate obtained in step (D) with at least one excipient to obtain a compression mixture;
   (F) optionally compressing the compression mixture to obtain a comprimate, in particular tablet; and
   (G) optionally applying a coating on the comprimate.
34. Process for the preparation of an apixaban-containing extrudate according to item 33, wherein said polymer component is polyvinyl caprolactam-polyvinyl acetate-polyethylene glycol graft copolymer.
35. Process for the preparation of an apixaban-containing extrudate according to item 33, wherein said polymer component is copovidone.
36. Process for the preparation of an apixaban-containing extrudate according to any of the preceding items 33-35, wherein the mixture prepared in step (A) has a weight ratio between apixaban and said polymer component (apixaban : polymer component) which is 1:6 or less, preferably 1:9 or less, especially in the range from 1:6 to 1:11, further especially in the range from 1:6 to 1:9.
37. Process for the preparation of an apixaban-containing extrudate according to any of the preceding items 33-36, wherein hot melt extrusion is carried out at a temperature of at least 190°C, and wherein the mixture prepared in step (A) has a weight ratio between apixaban and said polymer component (apixaban : polymer component) which is 1:9 or less, especially in the range from 1:9 to 1:11.
38. Process for the preparation of an apixaban-containing extrudate according to any of the preceding items 33-37, wherein hot melt extrusion is carried out at a temperature of at least 210°C, and wherein the mixture prepared in step (A) has a weight ratio between apixaban and said polymer component (apixaban : polymer component) which is 1:6 or less, preferably 1:9 or less, especially in the range from 1:6 to 1:11, further especially in the range from 1:6 to 1:9.
39. Process for the preparation of an apixaban-containing extrudate according to any of the preceding items 33-38, wherein the apixaban present in the extrudate obtained in step (B) consists of amorphous form of apixaban or comprises at least 80 wt.-%, preferably at least 95 wt.% of amorphous form of apixaban, based on the total weight of apixaban which is present in the extrudate.
40. Process for the preparation of an apixaban-containing extrudate according to any of the preceding items 33-38, wherein the apixaban present in the extrudate obtained in step (C) consists of amorphous form of apixaban or comprises at least 80 wt.-%, preferably at least 95 wt.% of amorphous form of apixaban, based on the total weight of apixaban which is present in the extrudate.

### Detailed description of the invention

The present invention relates to a pharmaceutical composition comprising apixaban, which pharmaceutical composition comprises or consists of one or more apixaban-containing granules, wherein each of said apixaban-containing granules comprises or consists of
(i) apixaban, and
(ii) hydroxyethyl cellulose.

Surprisingly, a pharmaceutical composition comprising one or more apixaban-containing granules, wherein each of said apixaban-containing granules comprises or consists of (i) apixaban, and (ii) hydroxyethyl cellulose provides highly advantageous dissolution properties and shows a highly advantageous apixaban bioavailabilty. Unexpectedly, apixaban-containing granules comprising or consisting of (i) apixaban, and (ii) hydroxyethyl cellulose exhibit superior dissolution and bioavailability, and provide a better dissolution profile than apixaban-containing granules comprising e.g. hydroxypropyl cellulose, hydroxypropylmethyl cellulose and PVP (polyvinylpyrrolidone). Furthermore, the dissolution of the pharmaceutical composition is insensitive to the presence of different amounts of hydroxyethyl cellulose in the pharmaceutical composition.

According to a first aspect, a pharmaceutical composition comprising apixaban is provided, said pharmaceutical composition comprising or consisting of one or more apixaban-containing granules, wherein each of said apixaban-containing granules comprises or consists of
(i) apixaban (optionally said apixaban being or comprising crystalline apixaban), and
(ii) hydroxyethyl cellulose.

Said crystalline apixaban can have average particle size between 0.1 and 200 µm, preferably between 1 and 80 µm, more preferably between 1-40 µm, most preferably 1-20 µm. The average particle size is determined by laser method using a Malvern Mastersizer.

According to a further aspect, a pharmaceutical composition comprising apixaban is provided, said pharmaceutical composition comprising or consisting of one or more apixaban-containing granules, wherein each of said apixaban-containing granules comprises or consists of apixaban-containing extrudate, said apixaban-containing extrudate comprising or consisting of apixaban, and a polymer component which is selected from polyvinyl caprolactam-polyvinyl acetate-polyethylene glycol graft copolymer, copovidone, poly(vinyl alcohol), and mixtures thereof.

In particular, a pharmaceutical composition comprising or consisting of one or more apixaban-containing granules is provided, wherein each of said apixaban-containing granules comprises or consists of
(α) apixaban-containing extrudate, in particular apixaban-containing hot-melt extrudate, comprising or consisting of
   (I.) apixaban, and
   (II.) a polymer component which is or comprises polymer selected from polyvinyl caprolactam-polyvinyl acetate-polyethylene glycol graft copolymer, copovidone, poly(vinyl alcohol), and mixtures thereof, wherein optionally said apixaban-containing extrudate, in particular apixaban-containing hot-melt extrudate, optionally has a weight ratio between apixaban and polymer component (apixaban : polymer component), which weight ratio can be 1:4 or less, especially can be 1:6 or less, especially can be in the range from 1:6 to 1:9, and
(β) optionally one or more intragranular excipients.

Apixaban is a known active agent, and can be prepared according to any of the processes described in the prior art. For example, apixaban can be prepared as disclosed in U.S. Patent No. 6,967,208. The term "apixaban" encompasses pharmaceutically acceptable salts, hydrates and/or solvates thereof.

Hydroxyethyl cellulose (HEC) is a known commercially available compound. Hydroxyethyl cellulose is a partially substituted poly(hydroxyethyl)ether of cellulose. HEC can be a non-ionic, water-soluble polymer. HEC is described in detail in U.S. Pharmacopeia USP32-N27, as well as in the textbook "Handbook of Pharmaceutical Excipients", Sixth ed., edited by R.C. Rowe et al., Pharmaceutical Press, ISBN 978 0 85369 792 3, in particular pages 311-314). Hydroxyethyl cellulose is available in a range of viscosity types. Hydroxyethyl cellulose having any viscosity type may be used for preparing the pharmaceutical compositions of the present invention.

The skilled person may choose the degree of substitution and the molecular weight of hydroxyethyl cellulose on the basis of the general knowledge in the art. The molecular weight of the hydroxyethyl cellulose can be e.g. in the range of about 4000 to about 1 600 000 g/mol, in particular of about 10 000 to about 1 350 000 g/mol.

The hydroxyethyl cellulose can have a viscosity in the range from 2 to 20 000 mPa·s, preferably 7 to 15000 mPa·s, most preferably from 60 to 5100 mPa·s, especially 70 to 450 mPa·s, in a 2 weight percent aqueous solution at 20°C (especially at 20°C, and a pressure of 101 325 Pa). Viscosities may be measured by conventional methods, for example, by measuring the viscosity of an aqueous solution of the polymers at the desired concentration in a device for determining viscosity (e.g. Ubbelohde capillary tubes) at the specified temperature. In particular, viscosity may be determined according to European Pharmacopoeia 9.2, in particular according to chapter 2.2.9 "Capillary viscometer method".

The weight average molecular weight of hydroxyethyl cellulose can be in the range 80,000 - 1,350,000 Da, especially in the range 89,000 - 350,000 Da.

The term "one or more apixaban-containing granules" can be preferably two or more, 3 or more, 4 or more, 10 or more, and can be e.g. 1 to 100 or 4 to 20, apixaban-containing granules.

A pharmaceutical composition comprising or consisting of one or more apixaban-containing granules, wherein each of said apixaban-containing granules comprises or consists of (i) apixaban, and (ii) hydroxyethyl cellulose, can exhibit dissolution properties that more than 70% of apixaban is dissolved in 22 minutes, especially 20 minutes in 900 ml of a buffer, which is a 50 mM sodium phosphate buffer having pH 6.8 and containing 0.05 wt.-% SDS (sodium dodecyl sulfate). The dissolution testing can be performed according to conventional methods known to the skilled person, for example at a temperature of 37°C. In a preferred embodiment of the present invention the dissolution testing is performed at 37°C using USP Apparatus 2 (paddles) method at a rotation speed of 75 rpm.

The one or more apixaban-containing granules can be prepared by any procedure known in the art, including but not limited to granulation, such as e.g. wet granulation, moisture-activated granulation, dry granulation, melt granulation, extrusion, especially hot melt extrusion, and combinations thereof. Optionally, the one or more apixaban-containing granules comprising HEC can be prepared by wet granulation.

The one or more apixaban-containing granules comprising HEC can be granules which are not prepared by dry granulation. In one embodiment, the one or more apixaban-containing granules comprising HEC can be granules which are not prepared by melt granulation.

In particular, each of the one or more apixaban-containing granules comprising HEC and apixaban can be prepared by wet granulation. Wet granulation provides particularly advantageous granules comprising HEC and apixaban. Especially, the one or more apixaban-containing granules comprising HEC and apixaban can be prepared by wet granulation in the presence of a granulation fluid comprising or consisting of water.

Further in particular, apixaban-containing granules comprising apixaban-containing extrudate can be prepared as described in more detail below, e.g. by compressing a mixture comprising apixaban-containing extrudate. Apixaban-containing extrudate can be in particular prepared by melt extrusion, especially hot melt extrusion. Melt extrusion, in particular hot melt extrusion, provides particularly advantageous extrudates and granules. Especially, the one or more apixaban-containing granules can be prepared by melt extrusion. Melt extrusion can be in particular a procedure known in the art as "Hot melt extrusion".

In particular, in a pharmaceutical composition comprising one or more apixaban-containing granules, each of said apixaban-containing granules (which comprise both HEC and apixaban) can have a weight ratio of apixaban : hydroxyethyl cellulose which is in the range from 1:10 to 10:1, preferably in the range from 1:5 to 5:1, more preferably in the range from 1:2 to 2:1, especially in the range from 1:1.6 to 1.6:1, or especially in the range from 1:7 to 1:4, particularly in the range from 1:1.65 to 1:1.55.

The pharmaceutical composition can comprise one or more further excipients in addition to apixaban, hydroxyethyl cellulose. The pharmaceutical composition can comprise in addition to apixaban, hydroxyethyl cellulose one or more further excipients, e.g. 1 or 2 or 3 or 4 or 5 further excipient(s), selected from the group of diluents, binders, disintegrants, lubricants, glidants, surfactants, and antitacking agents.

Furthermore, the one or more apixaban-containing granules can comprise one or more further excipients in addition to apixaban, hydroxyethyl cellulose. The one or more apixaban-containing granules can comprise in addition to apixaban, hydroxyethyl cellulose one or more further excipients, e.g. 1 or 2 or 3 or 4 or 5 further excipient(s), selected from the group of diluents, binders, disintegrants, lubricants, glidants, surfactants, and antitacking agents. Excipients used for the preparation of pharmaceutical compositions of the present invention can be pharmaceutically acceptable excipients, i.e. excipients having a quality appropriate for mammal use.

Excipients can have multiple functions, i.e. one excipient can be used as a binder/diluent, etc. Other and further excipients with different functions, e.g. polymers, pH modifiers, antioxidants, surfactants etc. can be included in the pharmaceutical compositions of the present invention (especially in the apixaban-containing granules), or one or more excipients with different functions can be excluded from the composition.

Each of the one or more apixaban-containing granules can comprise or can consist of
a) apixaban, hydroxyethyl cellulose, and a diluent, or
b) apixaban, hydroxyethyl cellulose, and a disintegrant, or
c) apixaban, hydroxyethyl cellulose, and a surfactant, or
d) apixaban, hydroxyethyl cellulose, a diluent, and a disintegrant, or
e) apixaban, hydroxyethyl cellulose, a diluent, and a surfactant.

In particular, a pharmaceutical composition comprising one or more apixaban-containing granules can be a pharmaceutical composition, wherein each of said apixaban-containing granules comprises or consists of apixaban, hydroxyethyl cellulose, a diluent, a disintegrant and a surfactant.

A pharmaceutical composition comprising one or more apixaban-containing granules can furthermore comprise at least one further granule. Said at least one further granule can comprise apixaban or can be free of apixaban.

The pharmaceutical composition of the present invention can be in solid dosage form such as a tablet, especially a coated tablet.

The pharmaceutical composition of the present invention can be a unit dosage form. The term "unit dosage form" refers to physically discrete unit(s) suitable as unitary dosages for human subjects and other mammals, each unit containing a predetermined quantity of apixaban calculated to produce the desired therapeutic effect, in association with at least one suitable excipient. In one embodiment, an immediate release composition of unit dosage form is preferred.

The pharmaceutical composition comprising one or more apixaban-containing granules can comprise:
(A) one or more apixaban-containing granules, wherein each of said apixaban-containing granules comprises or consists of
   (i) apixaban (optionally said apixaban being or comprising crystalline apixaban), and
   (ii) hydroxyethyl cellulose, and
(B) one or more extragranular excipients, wherein preferably said one or more extragranular excipients comprise a disintegrant.

Furthermore, a pharmaceutical composition comprising one or more apixaban-containing granules can be a pharmaceutical composition, wherein each of said apixaban-containing granules comprises or consists of apixaban, hydroxyethyl cellulose, and a diluent and has a weight ratio of apixaban : diluent which is in the range from 1:10 to 1:60, preferably from 1:20 to 1:50, more preferably from 1:30 to 1:40, especially from 1:34 to 1:38.

Moreover, a pharmaceutical composition comprising one or more apixaban-containing granules can be a pharmaceutical composition, wherein each of said apixaban-containing granules comprises or consists of apixaban, hydroxyethyl cellulose, and a diluent and has a weight ratio of hydroxyethyl cellulose : diluent which is in the range from 1:10 to 1:60, preferably from 1:20 to 1:50, more preferably from 1:30 to 1:40, especially from 1:34 to 1:38.

The pharmaceutical composition of the present invention may comprise diluent. In a pharmaceutical composition comprising one or more apixaban-containing granules and one or more extragranular excipients, the granulate may comprise diluent, or the one or more extragranular excipients may comprise diluent, or both granulate and the one or more extragranular excipients may comprise diluent.

The term diluent may refer to one or more diluents, and thus encompasses one diluent, as well as a mixture of diluents.

The diluent can be or can comprise diluent selected from the group consisting of carbohydrates or its derivatives, such as lactose, e.g. lactose monohydrate, anhydrous lactose, spray dried and/or granulated lactose, sucrose, fructose, dextrates, saccharose, raffinose, trehalose, fructose and mixtures thereof, dextrin, sugar alcohols such as xylitol, mannitol, maltitol, isomalt, sorbitol, celluloses, in particular powdered cellulose, microcrystalline cellulose, silicified microcrystalline cellulose, starch and derivatives thereof, in particular low moisture starch, starch such as maize starch, potato starch, rice starch, tapioca starch or wheat starch, pregelatinised starch, low moisture pregelatinised starch, magnesium aluminometasilicate, calcium salts of phosphoric acid, such as calcium hydrogen phosphate (in anhydrous or hydrate form), alkali carbonates, earth alkali carbonates, alkali hydrogencarbonates, earth alkali hydrogencarbonates, such as calcium carbonate, sodium carbonate or potassium carbonate or calcium hydrogencarbonate, sodium hydrogencarbonate or potassium hydrogencarbonate, and calcium lactate, and mixtures thereof.

Especially, the diluent can be or can comprise diluent selected from the group consisting of lactose, cellulose, and mixtures thereof, optionally the diluent can be a mixture comprising or consisting of lactose monohydrate and microcrystalline cellulose.

The pharmaceutical composition of the present invention can furthermore comprise disintegrant. In a pharmaceutical composition comprising one or more apixaban-containing granules and one or more extragranular excipients, each one of the one or more apixaban-containing granules may comprise disintegrant, or the one or more extragranular excipients may comprise disintegrant, or the one or more extragranular excipients and each one of the one or more apixaban-containing granules and may comprise disintegrant. In the present application, the term disintegrant may refer to one or more disintegrants, and thus encompasses one disintegrant, as well as a mixture of disintegrants. The disintegrant can be or can comprise disintegrant selected from crospovidone, starch such as maize starch, potato starch, rice starch, tapioca starch or wheat starch, pregelatinised starch, sodium starch glycolate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), polacrilin potassium, hydroxypropylcellulose, in particular low substituted hydroxypropylcellulose, sodium and/or calcium alginate, docusate sodium, methylcellulose, agar, guar gum, chitosan, alginic acid, and mixtures thereof. Preferably, disintegrant can be selected from cross-linked carboxymethylcelluloses, especially sodium croscarmellose and/or calcium croscarmellose), and mixtures thereof. More preferably, the apixaban-containing granules comprise sodium croscarmellose.

Sodium croscarmellose can be in particular the sodium salt of a cross-linked, partly O-carboxymethylated cellulose, and can be also known under the name "Cellulose, carboxymethyl ether, sodium salt, crosslinked". The terms "Sodium croscarmellose" and "Croscarmellose Sodium" are used interchangeably herein. Especially, sodium croscarmellose can be used for preparing the pharmaceutical composition of the present invention which is sodium croscarmellose, as defined in European Pharmacopoeia 6.0, chapter "Croscarmellose Sodium".

The pharmaceutical composition of the present invention can furthermore comprise lubricant. In particular, each of the one or more apixaban-containing granules can furthermore comprise lubricant. In a pharmaceutical composition comprising one or more apixaban-containing granules and one or more extragranular excipients, each one of the one or more apixaban-containing granules may comprise lubricant, and/or the one or more extragranular excipients may comprise lubricant. In the present application, the term lubricant may refer to one or more lubricants, and thus encompasses one lubricant, as well as a mixture of lubricants. The lubricant can be selected from the group of alkali alkylsulfates having 10 to 20 carbon atoms, preferably with 12 to 16 carbonatoms, such as sodium lauryl sulfate, metal salts of fatty acids having 12 to 20 carbon atoms such as magnesium stearate, calcium stearate, aluminium stearate or zinc stearate, magnesium palmitate and magnesium oleate, fatty acids having 12 to 20 carbon atoms such as stearic acid, palmitic acid and oleic acid, hydrogenated vegetable oil, hydrogenated castor oil, talc, meads wax or spermaceti, boric acid, sodium stearylfumarate, macrogols, and mixtures thereof.

Preferably, the apixaban-containing granules can comprise at least one lubricant selected from alkali metal salts of fatty acids with 10 to 20 carbon atoms, preferably with 12 to 16 carbonatoms, and mixtures thereof. More preferably, the apixaban-containing granules comprise at least one lubricant selected from the group of alkali alkylsulfates having 10 to 20 carbon atoms, preferably with 12 to 16 carbon atoms. An alkali alkylsufate can be in particular a compound of formula H₃C-(CH₂)ₙ-SO₄⁻Na⁺, wherein is n = 10 to 20, preferably 12 to 16.

The lubricant can be selected from the group of alkali alkylsulfates having 10 to 20 carbon atoms, preferably with 12 to 16 carbonatoms, such as sodium lauryl sulfate, metal (earth alkali) salts of fatty acids having 12 to 20 carbon atoms such as magnesium stearate, calcium stearate, aluminum stearate or zinc stearate, magnesium palmitate and magnesium oleate, fatty acids having 12 to 20 carbon atoms such as stearic acid, palmitic acid and oleic acid, hydrogenated vegetable oil, hydrogenated castor oil, talc, meads wax or spermaceti, boric acid, sodium stearylfumarate, macrogols, and mixtures thereof.

The pharmaceutical composition of the present invention and/or each of the one or more apixaban-containing granules can furthermore comprise one or more glidants, selected from colloidal silica, talc and magnesium trisilicate, preferably colloidal silica.

The pharmaceutical composition of the present invention, especially the apixaban-containing granules comprising HEC, can furthermore comprise binder other than hydroxyethyl cellulose. In a preferred embodiment, the apixaban-containing granules (especially those granules comprising apixaban and HEC), and in particular the pharmaceutical composition of the present invention, can be free of binder other than hydroxyethyl cellulose. In an embodiment, the apixaban-containing granules (especially the apixaban-containing granules comprising apixaban and HEC), and in particular the pharmaceutical composition of the present invention, can be free of binder selected from the group consisting of povidone (polyvinylpyrrolidone), copovidone (vinylpyrrolidone-vinyl copolymer), hydroxymethylcellulose, hydroxyethylcellulose, hydroxypropylcellulose, polymethacrylates, and mixtures therof, or can contain not more than 1wt.-% (preferably not more than 0,4 wt.-%) of one of povidone (polyvinylpyrrolidone), copovidone (vinylpyrrolidone-vinyl copolymer), hydroxymethylcellulose, hydroxyethylcellulose, hydroxypropylcellulose, polymethacrylates, based on the total weight of the apixaban-containing granules.

In a pharmaceutical composition comprising one or more apixaban-containing granules and optionally at least one extragranular excipient, each of said one or more apixaban-containing granules may comprise glidant, and/or the one or more extragranular excipients may comprise glidant.

The pharmaceutical composition of the present invention can furthermore comprise one or more antitacking agents, selected from talc, glyceryl monostearate and stearic acid, preferably stearic acid. In a pharmaceutical composition comprising one or more apixaban-containing granules and one or more extragranular excipients, the one or more apixaban-containing granules may comprise antitacking agent, or the one or more extragranular excipients may comprise antitacking agent, or both the one or more extragranular excipients and the one or more apixaban-containing granules may comprise antitacking agent.

In one embodiment, the pharmaceutical composition comprising one or more apixaban-containing granules can comprise:
(A) one or more apixaban-containing granules, wherein each of said apixaban-containing granules comprises or consists of
   (i) apixaban (optionally said apixaban being or comprising crystalline apixaban), and
   (ii) hydroxyethyl cellulose, and
(B) one or more extragranular excipients, wherein said one or more extragranular excipients comprise a disintegrant, and optionally a lubricant. In particular, the pharmaceutical composition can be a comprimate, especially a tablet. The one or more extragranular excipients can comprise at least one of a disintegrant and a lubricant. Preferably, the one or more extragranular excipients can comprise a disintegrant and a lubricant.

In one embodiment, the pharmaceutical composition of the present invention can be a pharmaceutical composition comprising:
(A') one or more apixaban-containing granules, wherein each of said apixaban-containing granules comprises or consists of
   (α)apixaban-containing extrudate, in particular apixaban-containing hot-melt extrudate, comprising or consisting of
      (I.) apixaban, and
      (II.) a polymer component which is or comprises polymer selected from polyvinyl caprolactam-polyvinyl acetate-polyethylene glycol graft copolymer, copovidone, poly(vinyl alcohol), and mixtures thereof, and
   (β) optionally one or more intragranular excipients, and
(B) one or more extragranular excipients, wherein preferably said one or more extragranular excipients comprise a disintegrant.

In particular, the pharmaceutical composition can be a comprimate, especially a tablet. Apixaban-containing extrudate comprising or consisting of apixaban, and a polymer component which is or comprises polymer selected from polyvinyl caprolactam-polyvinyl acetate-polyethylene glycol graft copolymer, copovidone, poly(vinyl alcohol), is described in more detail below.

The one or more intragranular excipients can comprise lubricant. The one or more extragranular excipients can comprise lubricant. Lubricant can be selected from the group of alkali alkylsulfates having 10 to 22 carbon atoms, preferably with 12 to 16 carbonatoms, such as sodium lauryl sulfate, metal (especially earth alkali) salts of fatty acids having 12 to 20 carbon atoms such as magnesium stearate, calcium stearate, aluminium stearate or zinc stearate, magnesium palmitate and magnesium oleate, fatty acids having 12 to 20 carbon atoms such as stearic acid, palmitic acid and oleic acid, hydrogenated vegetable oil, hydrogenated castor oil, talc, meads wax or spermaceti, boric acid, sodium stearylfumarate, macrogols, and mixtures thereof. In particular, the one or more extragranular excipients can comprise lubricant which can be selected from the group of metal salts (especially earth alkali salts) of fatty acids having 12 to 20 carbon atoms such as magnesium stearate, calcium stearate, aluminium stearate or zinc stearate, magnesium palmitate and magnesium oleate.

The one or more intragranular excipients can comprise disintegrant. The one or more extragranular excipients can comprise disintegrant. Disintegrant can be selected from crospovidone, starch such as maize starch, potato starch, rice starch, tapioca starch or wheat starch, pregelatinised starch, sodium starch glycolate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), polacrilin potassium, hydroxypropylcellulose, in particular low substituted hydroxypropylcellulose, sodium and/or calcium alginate, docusate sodium, methylcellulose, agar, guar gum, chitosan, alginic acid, and mixtures thereof. Preferably, the one or more extragranular excipients can comprise disintegrant which can be selected from cross-linked carboxymethylcelluloses, especially sodium croscarmellose and/or calcium croscarmellose), and mixtures thereof. More preferably, the apixaban-containing granules comprise sodium croscarmellose.

The pharmaceutical composition comprising one or more apixaban-containing granules can be a pharmaceutical composition wherein each of said apixaban-containing granules comprise:
apixaban in an amount of 0.7-30wt.%, preferably 1-15wt.%, more preferably 1.5-9wt.%, even more preferably 2-5wt.%, based on the total weight of the one or more apixaban-containing granules (optionally said apixaban being or comprising crystalline apixaban),
hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
optionally diluent in an amount of 50-99wt.%, preferably 70-97wt.%, more preferably 85-95wt.%, based on the total weight of the one or more apixaban-containing granules, optionally disintegrant in an amount of 0.5-10wt.%, preferably 1-8wt.%, more preferably 1.5-6wt.%, even more preferably 2-4wt.%, based on the total weight of the one or more apixaban-containing granules,
optionally surfactant in an amount of 0.1-5wt.%, preferably 0.5-2wt.%, more preferably 0.7-1.8wt.%, based on the total weight of the one or more apixaban-containing granules, and optionally one or more further excipients.

The total sum of the components of each of said apixaban-containing granules of the present invention can be 100%.

Especially, the pharmaceutical composition comprising one or more apixaban-containing granules can be a pharmaceutical composition wherein each of said apixaban-containing granules comprise:
apixaban in an amount of 0.7-30wt.%, preferably 1-15wt.%, more preferably 1.5-9wt.%, even more preferably 2-5wt.%, based on the total weight of the one or more apixaban-containing granules (optionally said apixaban being or comprising crystalline apixaban),
hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
diluent in an amount of 50-99wt.%, preferably 70-97wt.%, more preferably 85-95wt.%, based on the total weight of the one or more apixaban-containing granules,
optionally disintegrant in an amount of 0.5-10wt.%, preferably 1-8wt.%, more preferably 1.5-6wt.%, even more preferably 2-4wt.%, based on the total weight of the one or more apixaban-containing granules,
optionally surfactant in an amount of 0.1-5wt.%, preferably 0.5-2wt.%, more preferably 0.7-1.8wt.%, based on the total weight of the one or more apixaban-containing granules, and optionally one or more further excipients.

The total sum of the components of each of said apixaban-containing granules of the present invention can be 100%.

Further especially, the pharmaceutical composition comprising one or more apixaban-containing granules can be a pharmaceutical composition wherein each of said apixaban-containing granules comprise:
apixaban in an amount of 0.7-30wt.%, preferably 1-15wt.%, more preferably 1.5-9wt.%, even more preferably 2-5wt.%, based on the total weight of the one or more apixaban-containing granules (optionally said apixaban being or comprising crystalline apixaban),
hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
diluent in an amount of 50-99wt.%, preferably 70-97wt.%, more preferably 85-95wt.%, based on the total weight of the one or more apixaban-containing granules,
optionally disintegrant in an amount of 0.5-10wt.%, preferably 1-8wt.%, more preferably 1.5-6wt.%, even more preferably 2-4wt.%, based on the total weight of the one or more apixaban-containing granules,
surfactant in an amount of 0.1-5wt.%, preferably 0.5-2wt.%, more preferably 0.7-1.8wt.%, based on the total weight of the one or more apixaban-containing granules, and optionally one or more further excipients.

The total sum of the components of each of said apixaban-containing granules of the present invention can be 100%.

The pharmaceutical composition comprising one or more apixaban-containing granules can be a pharmaceutical composition, wherein each of said apixaban-containing granules comprises or consists of:
- apixaban in an amount of 0.7-30wt.%, preferably 1-15wt.%, more preferably 1.5-9wt.%, even more preferably 2-5wt.%, based on the total weight of the one or more apixaban-containing granules,
- hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
- lactose, in particular lactose monohydrate, in an amount of 25-60wt.%, preferably 38-55wt.%, more preferably 44-51wt.%, based on the total weight of the one or more apixaban-containing granules,
- microcrystalline cellulose in an amount of 25-55wt.%, preferably 38-49wt.%, more preferably 40-48wt.%, based on the total weight of the one or more apixaban-containing granules,
- sodium croscarmellose in an amount of 0.5-10wt.%, preferably 1-8wt.%, more preferably 1.5-6wt.%, even more preferably 2-4wt.%, based on the total weight of the one or more apixaban-containing granules,
- sodium lauryl sulfate in an amount of 0.1-5wt.%, preferably 0.5-2wt.%, more preferably 0.7-1.8wt.%, based on the total weight of the one or more apixaban-containing granules, and
- optionally one or more further excipients.

The total sum of the components of each of said apixaban-containing granules of the present invention can be 100%.

The pharmaceutical composition comprising one or more apixaban-containing granules can comprise:
one or more apixaban-containing granules (said one or more apixaban-containing granules being apixaban-containing granules comprising HEC, in particular as disclosed herein) in an amount of 30-99wt.%, preferably in an amount of 70-98wt%, more preferably in an amount of 85-97wt.%, especially in an amount of 90-95wt.%, based on the total weight of the pharmaceutical composition, and
one or more extragranular excipients, the total weight of extragranular excipients being in an amount of 1-70wt%, preferably in an amount of 2-30wt.%, more preferably in an amount of 3-15wt.%, especially in an amount of 5-10wt.%, based on the total weight of the pharmaceutical composition.

The total sum of the components of a composition of the present invention can be 100%.

According to another aspect, there is provided a process for preparing a pharmaceutical composition comprising apixaban, in particular pharmaceutical composition comprising one or more apixaban-containing granules, said process comprising
(i) preparing a granulate comprising apixaban and hydroxyethyl cellulose, preferably by wet granulation;
(ii) optionally drying the granulate obtained from step (i),
(iii) optionally sieving and/or milling the granulate obtained from step (i) or the optionally dried granules obtained from step (ii),
(iv) optionally combining the granulate obtained from step (i) or the optionally dried granules obtained from step (ii) or the optionally sieved and/or milled granules obtained from optional step (iii) with at least one excipient to obtain a compression mixture;
(v) optionally compressing the compression mixture to obtain a comprimate, in particular tablet; and
(vi) optionally applying a coating on the comprimate;
optionally, the pharmaceutical composition comprising apixaban, in particular the pharmaceutical composition comprising one or more apixaban-containing granules can be a pharmaceutical composition for oral administration. Optionally, the apixaban used for preparing the granulate being or comprising crystalline apixaban.

In particular, step i) of the process for preparing a pharmaceutical composition comprising apixaban can be
i) preparing a granulate comprising apixaban and hydroxyethyl cellulose by subjecting a mixture comprising apixaban and hydroxyethyl cellulose to wet granulation in the presence of a granulation fluid comprising a solvent, wherein the solvent is or comprises water.

Furthermore, step (i) of preparing a granulate comprising apixaban can comprise:
(I) preparing a mixture comprising apixaban, hydroxyethyl cellulose and optionally one or more further excipients;
(II) granulating the mixture obtained from step (I) in the presence of a granulation fluid comprising a solvent, said solvent preferably being or comprising water.

Granulating a mixture comprising apixaban, hydroxyethyl cellulose and optionally one or more further excipients in the presence of a granulation fluid can be in particular contacting said mixture comprising apixaban, hydroxyethyl cellulose and optionally one or more further excipients with granulation fluid, especially spraying granulation fluid on and optionally in the mixture comprising apixaban, hydroxyethyl cellulose and optionally one or more further excipients.

Step (i) of preparing a granulate comprising apixaban can be step (i) of preparing a granulate comprising or consisting of
a) apixaban, hydroxyethyl cellulose, and a diluent, or
b) apixaban, hydroxyethyl cellulose, and a disintegrant, or
c) apixaban, hydroxyethyl cellulose, and a surfactant, or
d) apixaban, hydroxyethyl cellulose, a diluent, and a disintegrant, or
e) apixaban, hydroxyethyl cellulose, a diluent, and a surfactant, or
f) apixaban, hydroxyethyl cellulose, a diluent, a disintegrant and a surfactant,
preferably by wet granulation or melt granulation, especially wet granulation.

In one embodiment, a process for preparing a pharmaceutical composition comprising apixaban, in particular pharmaceutical composition comprising one or more apixaban-containing granules, can comprise
(i) preparing a granulate comprising apixaban and hydroxyethyl cellulose by wet granulation;
(ii) drying the granulate obtained from step (i),
(iii) optionally sieving and/or milling the dried granules obtained from step (ii),
(iv) combining the dried granules obtained from step (ii) or the optionally sieved and/or milled granules obtained from optional step (iii) with at least one excipient to obtain a compression mixture;
(v) compressing the compression mixture to obtain a comprimate, in particular tablet; and
(vi) optionally applying a coating on the comprimate.

According to another aspect, the present invention provides a pharmaceutical composition comprising one or more apixaban-containing granules as defined herein, for use as a medicament.

According to another aspect, a pharmaceutical composition of the present invention can be for use as a medicament, especially can be for use in treating or preventing a thromboembolic disorder or disease, in particular a venous thromboembolic disorder or disease.

Wet granulation procedures are known to the skilled person; in particular, wet granulation can be a granulation comprising contacting a compound or mixture to be granulated (during and/or before granulation) with a granulation fluid, especially with granulation fluid comprising or consisting of water. Preferably, the granulation fluid comprises water as the main component, the main component being the component which is present in the highest amount of weight in the granulation fluid, when comparing the amounts of weight of all components present in the granulation fluid.

In particular, the process for preparing a pharmaceutical composition comprising apixaban-containing granules can comprise mixing apixaban with hydroxyethyl cellulose, sodium lauryl sulfate and diluent (e.g. lactose). Optionally, the mixture can be sieved through a mesh, e.g. with screen size 30#. The so obtained mixture (e.g. triturate of apixaban) can then be mixed with diluent (e.g. microcrystalline celullose and lactose) to obtain a granulation mixture.

The so obtained granulating mixture can then be optionally subjected to dry blending. Water can then be sprayed on the granulation mixture (e.g. at the rate of in the range of from 40-200 g/min), preferably until sufficient agglomeration was obtained. After optional homogenization of the granulate, the granulate can be dried (e.g. in a fluid-bed at inlet air temperature of at least 30 °C), for example until less than 3 % loss on drying (e.g. at 105 °C, during 5 min) was achieved. The dried granulate can then be sieved. Tableting mixture can be prepared by first mixing the granulate with disintegrant, preferably sodium croscarmellose, and then lubricant, e.g. magnesium stearate, can be added. Tableting can be performed for example on a rotary tablet press. Tablet cores can then be coated, for example using standard coating procedure until the desired mass gain was achieved.

The term "intragranularly" can refer to an intragranular phase, such as granules, wherein apixaban is granulated with an excipient or a mixture of excipients. The term "extragranularly" can relate to the additives added to the granulate, which additives can be excipient(s) with or without apixaban, preferably without apixaban.

In another embodiment, the pharmaceutical composition of the present invention can comprise apixaban having average particle size between 0.1 and 200 µm, optionally between 1 and 80 µm. The average particle size is determined by laser method using a Malvern Mastersizer.

The term "average particle size" as used herein refers to volume mean diameter of particles. The diameter and volume mean diameter can be determined by laser light scattering using e.g. a Malvern Matersizer Aparatus. Particle sizes are determined by measuring the angular distribution of laser light scattered by a homogeneous suspension of particles. The particles to be subjected to the particle size measurement are first suspended in appropriate non-polar dispersant and then subjected to a size determination in a Malvern Mastersizer instrument.

A preferred pharmaceutical composition of the present invention is a solid pharmaceutical composition. The composition can be in a form selected from the group of tablets, granules, granulates, minitablets, microtablets, coated tablets, coated minitablets, coated microtablets, pills, powders, lozenges, sachets, soft and hard gelatine capsules, suppositories, etc., and mixtures thereof. Tablets, minitablets, microtablets, coated tablets, coated minitablets, coated microtablets, pills, granules, granulates, powders can be filled into sachet(s), capsules, e.g. soft or hard gelatine capsules. The pharmaceutical composition of the present invention is preferably suitable for oral application. Thus, it is preferred that the pharmaceutical composition is an oral dosage form such as a tablet or a coated tablet. The pharmaceutical composition can also be a tablet core, granules or granulate.

The pharmaceutical composition of the present invention is preferably formulated in a unit dosage form, each unit dosage form containing about 1 to about 100 mg of apixaban, more preferably about 2 to about 8 mg of apixaban, most preferably 2.5 mg or 5 mg. The term "apixaban" can relate to apixaban in crystalline form, and in amorphous form. Apixaban can be selected from apixaban in crystalline form, preferably Form N-1 or Form H2-2, and amorphous form, and mixtures thereof.

Form N-1 (neat) and Form H2-2 (hydrate) of apixaban may be characterized by unit cell parameters substantially equal to the following shown in the following Table A, published in WO 2011/106478 A2:

**TABLE A**

| Form | N-1 | H2-2 |
|---|---|---|
| Solvate | None | Dihydrate |
| T | +22 | +22 |
| a(Å) | 10.233(1) | 6.193(1) |
| b(Å) | 13.852(1) | 30.523(1) |
| c(Å) | 15.806(1) | 13.046(1) |
| α,° | 90 | 90 |
| β,° | 92.98(1) | 90.95(1) |
| γ,° | 90 | 90 |
| V(Å³) | 2237.4(5) | 2466.0(5) |
| Z' | 1 | 1 |
| Vm | 559 | 617 |
| SG | P2₁/n | P2₁/n |
| Dcalc | 1.364 | 1.335 |
| R | 0.05 | 0.09 |
| Sol.sites | None | 2 H₂O |

| | | |
|---|---|---|
| Z' is the number of molecules per asymmetric unit. T(°C) is the temperature for the crystallographic data. Vm = V(unit cell) / (ZZ') | | |

Furthermore, characteristic X-ray diffraction peak positions (degrees 2θ±0.1) at room temperature, based on a pattern (especially a high quality pattern) collected with a diffractometer (CuKα) (with a spinning capillary with 2θ calibrated with a NIST suitable standard), are provided in WO 2011/106478 A2 and in EP 2 538 925 B1 and are shown in Table B below for Form N-1 and H2-2, respectively. Thus, Form N-1 can be characterized by a X-ray diffraction pattern collected with a diffractometer (CuKα) showing the characteristic X-ray diffraction peak positions (degrees 2θ±0.1) shown in Table B below, and Form H2-2 can be characterized by a X-ray diffraction pattern collected with a diffractometer (CuKα) showing the characteristic X-ray diffraction peak positions (degrees 2θ±0.1) shown in Table B below:

**Table B**

| Form N-1 | Form H2-2 |
|---|---|
| 10.0 | 5.8 |
| 10.6 | 7.4 |
| 12.3 | 16.0 |
| 12.9 | 20.2 |
| 18.5 | 23.5 |
| 27.1 | 25.2 |

According to a further aspect, a pharmaceutical composition comprising apixaban is provided, said pharmaceutical composition comprising or consisting of apixaban-containing extrudate, in particular apixaban-containing hot-melt extrudate,
wherein said apixaban-containing extrudate comprises or consists of
(I.) apixaban, and
(II.) a polymer component which is or comprises polymer selected from polyvinyl caprolactam-polyvinyl acetate-polyethylene glycol graft copolymer, copovidone, poly(vinyl alcohol), and mixtures thereof, and
wherein said apixaban-containing extrudate, in particular apixaban-containing hot-melt extrudate, has a weight ratio between apixaban and polymer component (apixaban : polymer component), which weight ratio is 1:4 or less, especially is 1:6 or less, especially is in the range from 1:6 to 1:9,
wherein optionally said apixaban-containing extrudate, in particular apixaban-containing hot-melt extrudate, is obtainable from a process comprising extrusion, in particular hot melt extrusion, carried out at a temperature of at least 190°C, especially at a temperature in the range from 190°C to 230°C.

In particular, extrudate (especially melt extrudate, further especially hot melt extrudate) comprising or consisting of (I.) apixaban, and (II.) a polymer component which is or comprises polymer selected from polyvinyl caprolactam-polyvinyl acetate-polyethylene glycol graft copolymer, copovidone, poly(vinyl alcohol), and mixtures thereof, has advantageous properties, for example an advantageous solubility. Without wishing to be bound by theory, it is currently assumed that the combination of apixaban, and a polymer component which is or comprises polymer selected from polyvinyl caprolactam-polyvinyl acetate-polyethylene glycol graft copolymer, copovidone, poly(vinyl alcohol), and mixtures thereof, and hot melt extrusion process contributes to the formation of amorphous apixaban in the extrudate. In particular, apixaban forms different from amorphous apixaban can be transformed during hot melt extrusion into amorphous apixaban.

Furthermore, the apixaban-containing extrudate can comprise one or more further excipients in addition to apixaban, and a polymer component which is or comprises polymer selected from polyvinyl caprolactam-polyvinyl acetate-polyethylene glycol graft copolymer, copovidone, poly(vinyl alcohol), and mixtures thereof. The apixaban-containing extrudate can comprise in addition to apixaban, and the polymer component as defined above, one or more further excipients, e.g. 1 or 2 or 3 or 4 or 5 further excipient(s), selected from the group of diluents, binders, disintegrants, lubricants, glidants, surfactants, and antitacking agents.

Furthermore, an apixaban-containing granule can comprise one or more further excipients in addition to apixaban, and a polymer component. A granule (or granulate) comprising apixaban and a polymer component can comprise one or more further excipients, e.g. 1 or 2 or 3 or 4 or 5 further excipient(s), selected from the group of diluents, binders, disintegrants, lubricants, glidants, surfactants, and antitacking agents, as intragranular excipients.

In particular, a granule (or granulate) comprising apixaban and a polymer component can comprise as the one or more optionally present intragranular excipients diluent (e.g. diluent which can be or comprise diluent selected from the group consisting of lactose (in particular lactose monohydrate), cellulose (in particular microcrystalline cellulose), and mixtures thereof), disintegrant (e.g. sodium croscarmellose), optionally lubricant, and optionally surfactant, and optionally one or more further excipients.

A pharmaceutical composition comprising apixaban and a polymer component can furthermore comprise one or more further excipients, e.g. 1 or 2 or 3 or 4 or 5 further excipient(s), selected from the group of diluents, binders, disintegrants, lubricants, glidants, surfactants, and antitacking agents, as the one or more extragranular excipients. In particular, the pharmaceutical composition comprising apixaban and a polymer component can comprise as extragranular excipients optionally diluent (e.g. diluent which can be or comprise diluent selected from the group consisting of lactose (in particular lactose anhydrous), cellulose (in particular microcrystalline cellulose), colloidal silicon dioxide, and mixtures thereof), disintegrant (e.g. sodium croscarmellose), optionally lubricant, and optionally surfactant, and optionally one or more further excipients.

In particular, said polymer component can be polyvinyl caprolactam-polyvinyl acetate-polyethylene glycol graft copolymer.

A polyvinyl caprolactam-polyvinyl acetate-polyethylene glycol graft copolymer (PCA-PVA-PEG) can be a polymer comprising at least one caprolactam block, at least one polyvinyl acetate block, and at least one polyethylene glycol block, wherein at least one block branches from another of the type of blocks. For example, some PCA-PVA-PEGs may be represented by the structure below:

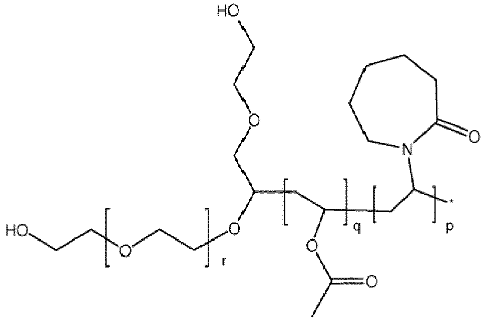

In the structure above, p may be about 10 to about 10,000, about 100 to about 900, about 100 to about 500, or about 500 to about 900.

In the structure above, q may be about 20 to about 20,000, about 150 to about 1500, about 200 to about 800, or about 800 to about 1500.

In the structure above, r may be about 30 to about 30,000, about 300 to about 3000, about 300 to about 1000, about 1000 to about 2000, or about 2000 to about 3000.

In particular, a PCA-PVA-PEG can have an average molecular weight (in particular an weight average molecular weight) of about 1,000 g/mol to about 5,000,000 g/mol, about 10,000 g/mol to about 500,000 g/mol, or about 90,000 g/mol to about 140,000 g/mol. Methods for determining the average molecular weight are known in the art, e.g. gel permeation chromatography can be used for determining the average molecular weight.

For example, PCA-PVA-PEG can be a polymer represented by CAS No. 402932-23-4. Especially, PCA-PVA-PEG can be SOLUPLUS®, available from BASF.

Especially, said polymer component can be copovidone.

Copovidone is a commercially available component and is known to the skilled person. Copovidone can be a synthetic, linear, random copolymer of N-vinyl-2-pyrrolidone (VP) and vinyl acetate (VA), wherein in particular the VA content can be nominally 40% (but may vary, for example between 35-41%). Especially, copovidone can be a copolymer of 1-ethenylpyrrolidin-2-one and ethenyl acetate, e.g. in the mass proportion 3:2. In particular, Copovidone can be copovidone as defined in European Pharmacopoeia, e.g. in European Pharmacopoeia 6.0.

In particular, said polymer component can be poly(vinyl alcohol) (PVA). Poly(vinyl alcohol) (PVA) can be a synthetic polymer, which can be prepared by polymerization of vinyl acetate and partial hydrolysis of the resulting esterified polymer.

Especially, polyvinyl alcohol can be polyvinyl alcohol as defined in European Pharmacopoeia (reference is in particular made to Ph. Eur. chapter entitled "POLY(VINYL ALCOHOL)"; Ph. Eur. can be especially Ph. Eur. as in force on 15.11.2018). PVA can have an ester value (e.g. as defined in Ph. Eur.) characterizing the degree of hydrolysis, which ester value is no greater than 280. PVA can have a mean relative molecular mass between 20000 and 150000 (e.g. as defined in Ph. Eur.). PVA can have the following formula:

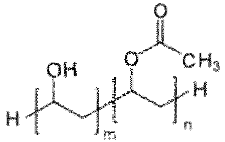

In particular, the indices m and n can be chosen such that m and n comply with: 0 ≤ n/m ≤ 0.35.

Polyvinyl alcohol (PVA) can be extrudable, in particular melt extrudable, especially hot melt extrudable PVA. Polyvinyl alcohol can be an extrudable polyvinyl alcohol (PVA) powder. Polyvinyl alcohol can be polyvinyl alcohol powder obtainable from milling, especially cryo-milling of polyvinyl alcohol.

PVA can be a polymer possessing both a glass transition temperature and a melting temperature. PVA can be a polymer having a melting temperature of 170°C or less. PVA can be preferably PVA 4 - 88. PVA is commercially available, and is for example marketed as Parteck® MXP by Merck KGaA.

In particular, the weight ratio apixaban : polymer component can be 1:6 or less, preferably 1:9 or less, e.g. 1:7 or less, especially in the range from 1:6 to 1:11, e.g. 1:7 to 1:11, further especially in the range from 1:6 to 1:9.

Especially, hot melt extrusion can be carried out at a temperature of at least 190°C.

In particular, hot melt extrusion can be carried out at a temperature of at least 190°C, and the weight ratio apixaban : polymer component can be 1:9 or less, especially in the range from 1:9 to 1:11.

Advantageously, hot melt extrusion can be carried out at a temperature of at least 210°C, and the weight ratio apixaban : polymer component be 1:6 or less, preferably 1:9 or less, e.g. 1:7 or less, especially in the range from 1:6 to 1:11, e.g. 1:7 to 1:11, further especially in the range from 1:6 to 1:9.

Each of said granules (optionally comprising polymer component) can comprise amorphous form of apixaban, optionally a mixture comprising amorphous form of apixaban and apixaban form N-1. Furthermore, each of said apixaban-containing granules comprising polymer component can be prepared by a process comprising or consisting of hot melt extrusion.

According to one embodiment, apixaban-containing extrudate can comprise or can consist of (I.) apixaban, and (II.) a polymer component which is or comprises poly(vinyl alcohol), and wherein said apixaban-containing extrudate has a weight ratio between apixaban and poly(vinyl alcohol) (apixaban : poly(vinyl alcohol)), which weight ratio is 1:4 or less, especially 1:6 or less, further especially in the range from 1:4 to 1:9, further especially in the range from 1:6 to 1:7, in particular about 1:6, wherein optionally said apixaban-containing extrudate is obtainable from a process comprising hot melt extrusion carried out at a temperature of at least 190°C, especially at a temperature in the range from 200°C to 220°C, further especially about 210°C.

Optionally, the apixaban-containing extrudate can be present in a mixture (especially a compressed mixture, e.g. a tablet) comprising one or more further excipients, e.g. 1 or 2 or 3 or 4 or 5 further excipient(s), selected from the group of diluents, binders, disintegrants, lubricants, glidants, surfactants, and antitacking agents.

In an embodiment, the pharmaceutical composition can be or comprise a mixture (especially a compressed mixture, e.g. a tablet) comprising (A.) apixaban-containing extrudate, especially apixaban-containing hot-melt extrudate, (especially as disclosed herein) and (B.) one or more further mixture components. These one or more further mixture components can be or comprise one or more further excipients, e.g. 1 or 2 or 3 or 4 or 5 further excipient(s), selected from the group of diluents, binders, disintegrants, lubricants, glidants, surfactants, and antitacking agents. In particular, the (B.) one or more further mixture components can comprise diluent (e.g. diluent which can be or comprise diluent selected from the group consisting of lactose (in particular lactose anhydrous), cellulose (in particular microcrystalline cellulose), and mixtures thereof), disintegrant (especially sodium croscarmellose), optionally lubricant (especially magnesium stearate), and optionally one or more further excipients. Optionally, said pharmaceutical composition (especially compressed mixture, e.g. tablet) can be coated with a tablet coating.

According to one embodiment, apixaban-containing extrudate can comprise or can consist of (I.) apixaban, and (II.) a polymer component which is or comprises polyvinyl caprolactam-polyvinyl acetate-polyethylene glycol graft copolymer (PCA-PVA-PEG), and wherein said apixaban-containing extrudate has a weight ratio between apixaban and PCA-PVA-PEG (apixaban : PCA-PVA-PEG), which weight ratio is 1:4 or less, especially 1:6 or less, further especially in the range from 1:6 to 1:9, further especially in the range from 1:6 to 1:7, in particular about 1:6, wherein optionally said apixaban-containing extrudate is obtainable from a process comprising hot melt extrusion carried out at a temperature in the range from 222°C to 232°C, further especially at a temperature in the range from 227°C to 231°C, especially about 230°C.

According to one embodiment, apixaban-containing extrudate can comprise or can consist of (I.) apixaban, and (II.) a polymer component which is or comprises copovidone, and wherein said apixaban-containing extrudate has a weight ratio between apixaban and copovidone (apixaban : copovidone), which weight ratio is 1:4 or less, especially 1:6 or less, further especially in the range from 1:6 to 1:9, further especially in the range from 1:5.5 to 1:7, especially about 1.6, wherein optionally said apixaban-containing extrudate is obtainable from a process comprising hot melt extrusion carried out at a temperature in the range from 200°C to 210°C.

According to one embodiment, apixaban-containing extrudate can comprise or can consist of (I.) apixaban, and (II.) a polymer component which is or comprises copovidone, and wherein said apixaban-containing extrudate has a weight ratio between apixaban and copovidone (apixaban : copovidone), which weight ratio is 1:9 or less, especially is in the range from 1:9 to 1:11, especially is in the range from 1:9 to 1:10, wherein optionally said apixaban-containing extrudate is obtainable from a process comprising hot melt extrusion carried out at a temperature of at least 190°C, preferably in the in the range from 190°C to 210°C.

Optionally, said apixaban-containing extrudate or said one or more apixaban-containing granules can comprise apixaban in amorphous form, preferably comprise at least 80 wt.-%, especially at least 95 wt.-%, further especially at least 99 wt.-% of the apixaban in amorphous form.

Said pharmaceutical composition can be for example a comprimate, especially a tablet, or can be filled into capsules or sachets, e.g. in the form of a powder.

The pharmaceutical composition (especially the composition comprising polymer component, in particular the composition comprising apixaban-containing extrudate) can comprise: one or more apixaban-containing granules (comprising polymer component, especially one or more apixaban-containing granules comprising or consisting of apixaban-containing extrudate comprising polymer component, in particular as disclosed herein), optionally in an amount of 30-99wt.%, preferably in an amount of 70-98wt%, more preferably in an amount of 85-97wt.%, especially in an amount of 90-97wt.%, based on the total weight of the pharmaceutical composition, and
one or more extragranular excipients, the total weight of extragranular excipients being optionally in an amount of 1-70wt%, preferably in an amount of 2-30wt.%, more preferably in an amount of 3-15wt.%, especially in an amount of 3-10wt.%, based on the total weight of the pharmaceutical composition. Said one or more extragranular excipients can in particular comprise disintegrant (e.g. sodium croscarmellose), and optionally one or more further excipients.

The composition comprising apixaban-containing extrudate does however not need to be further granulated according to one embodiment.

According to a further aspect, there is provided a process for the preparation of an apixaban-containing extrudate, and optionally for the preparation of a granulate or a comprimate, said apixaban-containing extrudate comprising or consisting of (I) apixaban, and (II) a polymer component which is or comprises polymer selected from polyvinyl caprolactam-polyvinyl acetate-polyethylene glycol graft copolymer, copovidone, poly(vinyl alcohol), and mixtures thereof,
which process comprises:
(A) preparing a mixture comprising (I) apixaban, and (II) a polymer component which is or comprises polymer selected from polyvinyl caprolactam-polyvinyl acetate-polyethylene glycol graft copolymer, copovidone, poly(vinyl alcohol), and mixtures thereof, optionally apixaban and said polymer component being present in said mixture in a weight ratio apixaban : polymer component which can be 1:4 or less, especially can be 1:6 or less, especially can be in the range from 1:6 to 1:9;
(B) subjecting the mixture obtained from step (A) to extrusion, especially hot melt extrusion, optionally the hot melt extrusion being carried out at a temperature of at least 190°C, further optionally at a temperature in the range of from 190°C to 230°C;
(C) optionally comminuting the apixaban-containing extrudate obtained in step (B) to obtain comminuted apixaban-containing extrudate;
(D) optionally converting the apixaban-containing extrudate obtained in step (B) or the comminuted apixaban-containing extrudate obtained in step (C) to form a granulate;
(E) optionally mixing the apixaban-containing extrudate obtained in step (B) or the comminuted apixaban-containing extrudate obtained in step (C) or the granulate obtained in step (D) with at least one excipient to obtain a compression mixture;
(F) optionally compressing the compression mixture to obtain a comprimate, in particular tablet; and
(G) optionally applying a coating on the comprimate.

The step of converting the apixaban-containing extrudate obtained in step (B) or the comminuted apixaban-containing extrudate obtained in step (C) to form a granulate can be or comprise: combining the apixaban-containing extrudate obtained in step (B) or the comminuted apixaban-containing extrudate obtained in step (C) with at least one excipient to obtain a extrudate-containing mixture, and compacting the extrudate-containing mixture to obtain a compacted extrudate-containing mixture, especially a granulate, e.g. in the form of a ribbon. Optionally, the compacted extrudate-containing mixture, especially the granulate, can be further comminuted.

The step of converting the apixaban-containing extrudate obtained in step (B) or the comminuted apixaban-containing extrudate obtained in step (C) to form a granulate can be or comprise: combining the apixaban-containing extrudate obtained in step (B) or the comminuted apixaban-containing extrudate obtained in step (C) with at least one excipient to obtain a extrudate-containing mixture, and subjecting the extrudate-containing mixture to wet granulation, e.g. using a granulation liquid comprising or consisting of a solvent selected from methanol, ethanol, propanol, water, and mixtures thereof.

The step of comminuting extrudate or of comminuting compressed extrudate-containing mixture, especially a granulate, may be carried out according to conventional milling methods or micronisation methods. For example, comminuting can be milling or milling and sieving. Comminuting can be e.g. carried out in an air jet mill, a hammer and screen mill, a fine impact mill, a ball mill or a vibrator mill or combinations thereof. Micronisation can be effected by known methods using an ultrasonic disintegrator, e.g. of the BRANSON Sonifier type, or by stirring a suspension with a high speed agitator, for example with a stirrer of the HOMOREX type.

Compaction may be carried out using a slugging or roller compaction technique. The preferred method of dry granulation can be roller compaction. Roller compaction is known in the art. Roller compaction (essentially) utilizes two rollers which roll towards each other. A hydraulic ram forces one of the rollers against the other roller to exert a compacting force against the ground particles fed into the roller compactor via a screw conveyor system. A compaction force of minimal 10 kN can be used. The comprimate can be in the form of a ribbon, e.g. in segments, depending on the surface of the rollers. The comprimate may be milled and/or sieved to produce the granulate.

The apixaban present in the extrudate obtained in step (B) can consist of amorphous form of apixaban or can comprise at least 80 wt.-%, preferably at least 95 wt.% of amorphous form of apixaban, based on the total weight of apixaban which is present in the extrudate.

The apixaban present in the extrudate obtained in step (C) can consist of amorphous form of apixaban or can comprise at least 80 wt.-%, preferably at least 95 wt.% of amorphous form of apixaban, based on the total weight of apixaban which is present in the extrudate.

Surprisingly, apixaban-containing extrudates obtained by processes involving Hot Melt Extrusion as disclosed herein, especially apixaban-containing extrudates obtained by a process for the preparation of an apixaban-containing extrudate according to the present invention, provide highly advantageous dissolution properties.

In one embodiment of the process for the preparation of an apixaban-containing extrudate, said polymer component can be polyvinyl caprolactam-polyvinyl acetate-polyethylene glycol graft copolymer. In one embodiment, said polymer component is copovidone. In one embodiment, said polymer component is poly(vinyl alcohol).

The mixture comprising apixaban, and a polymer component can be a mixture having a weight ratio apixaban : polymer component which weight ratio can be 1:4 or less, 1:6 or less, preferably 1:9 or less, especially in the range from 1:6 to 1:11, e.g. in the range from 1:7 to 1:11, further especially in the range from 1:6 to 1:9.

In the process for the preparation of an apixaban-containing extrudate, hot melt extrusion can be carried out at a temperature of at least 190°C, and the weight ratio apixaban : polymer component can be 1:9 or less, especially can be in the range from 1:9 to 1:11.

In particular, hot melt extrusion can be carried out at a temperature of at least 210°C, and the weight ratio apixaban : polymer component can be 1:6 or less, e.g. 1:7 or less, preferably 1:9 or less, especially can be in the range from 1:6 to 1:11, e.g. in the range from 1:7 to 1:11, further especially in the range from 1:6 to 1:9.

As starting material apixaban of any form can be used; for example, apixaban being or comprising apixaban Form N-1 (or apixaban being or comprising amorphous form and optionally Form N-1) can be used for preparing the mixture in step (A).

Hot melt extrusion for the preparation of an apixaban-containing extrudate can be carried out at a temperature of at least 190°C, for example at a temperature in the range of from 190°C to 230°C, optionally at a temperature in the range of from 200°C to 228°C, further optionally in the range of from 190°C to 228°C.

Furthermore, there is provided an apixaban-containing extrudate (and optionally a comprimate or granulate containing apixaban-containing extrudate), which is obtainable by a process for preparing an apixaban-containing extrudate or for preparing an apixaban-containing comprimate or granulate disclosed herein, especially by a process as defined in the claims annexed.

Pharmaceutical compositions of the present invention, especially pharmaceutical compositions of the present invention which are solid dosage forms, such as e.g. tablets, can be optionally coated with aqueous soluble film coating, having average thickness of at least 1 µm, measured by scanning electron microscopy (SEM) of crossection of coated solid dosage form. Optional film coating can comprise aqueous soluble polymer selected from cellulose ethers, such as hydroxypropylmethylcellulose (hypromellose) having viscosity of 2wt.-% aqueous solution less than 60 mPa·s, preferably less than 40 mPa·s and most preferably less than 20 mPa·s measured at 20°C. Other polymers that can be used for coating are selected from polyvinyl alcohol, povidone, sodium carboxymethyl cellulose, waxy materials, acrylic polymers, block polymer of polyvinyl alcohol and polyethylene glycol commercially available under trade name Kollicoat® IR and Kollicoat ® Protect. Optionally, other excipients can be used and are selected from lubricants, antitacking agents, pigments, colourant and/or plasticizers.

Typical cellulose ethers used in film coatings (which can be used for coating the pharmaceutical compositions of the present invention) are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

Commonly used plasticizers (which can be used for coating the pharmaceutical compositions of the present invention) can be categorized into three groups: polyols (glycerol, propylene glycol, macrogols), organic esters (phthalate esters, dibutyl sebacate, citrate esters, triacetin), oils/glycerides (castor oil, acetylated monoglycerides, fractionated coconut oil).

Commonly used lubricants and/or antitacking agents (which can be used for coating the pharmaceutical compositions of the present invention) can be selected from the group of metal salts of fatty acids with 12 to 20 carbon atoms such as magnesium stearate, calcium stearate, aluminium stearate or zinc stearate, magnesium palmitate and magnesium oleate, fatty acids with 12 to 20 carbon atoms such as stearic acid, palmitic acid and oleic acid, hydrogenated vegetable oil, hydrogenated castor oil, talc, meads wax or spermaceti, boric acid, sodium stearyl fumarate, and mixtures thereof.

Colourants/opacifiers (which can be used for coating the pharmaceutical compositions of the present invention) are classified into several groups: organic dyes and their lakes, inorganic colours, natural colours. Pigments can be selected from metal oxides such as iron or titanium oxides.

Combination of different materials from each group can be combined, in particular in defined ratios.

Film coating suspensions can be used as ready-to-make preparations which are available on the market. Film coating dispersion can be prepared by using different solvents (water, alcohols, ketones, esters, chlorinated hydrocarbons), preferably water.

A composition of coating suspension (calculated on dry material) is particularly preferred which comprises:
- 1-99% by weight of polymer, preferably 1-95% of polymer,
- 1-50% by weight of plasticizer, preferably 1-40% of plasticizer,
- 0.1-20% by weight of lubricant, preferably 1-10% of lubricant,
- 0.1-20% by weight of colourant/opacifier and/or pigment, preferably 0.1-10% of colourant/opacifier and or/pigment.

The composition of the coating layer of the pharmaceutical composition of the present invention preferably comprises at least one excipient selected from excipients with the function as defined of polymer and plasticizer. In another embodiment of the present invention, the composition of the coating layer of the present invention preferably comprises at least one excipient selected from excipients with the function as defined polymer, plasticizer, lubricant and colourant/opacifier.

If not indicated otherwise, all percentages ("%") given herein are wt. %. Excipients mentioned herein, in particular pH modifier, polymer, can be pharmaceutically acceptable excipients. The term "pharmaceutically acceptable" as used herein can in particular indicate that the "pharmaceutically acceptable" compound or composition is suitable for administration to a subject to achieve a treatment and/or prevention of a disease, of a disorder or of a condition, without unduly deleterious side effects in light of the necessity of the treatment. The term "therapeutically effective amount" as used herein can in particular indicate an amount sufficient to ameliorate, reverse, stabilize, slow and/or delay progression of a disease, a disorder or a condition.

According to one embodiment, the pharmaceutical composition of the present invention is a coated comprimate comprising a comprimate and a coating coated on the comprimate, (e.g. a coated tablet comprising a tablet core and a coating coated on the tablet core), wherein the comprimate comprises: one or more apixaban-containing granules in an amount of 30-99wt.%, preferably in an amount of 70-98wt%, more preferably in an amount of 85-97wt.%, especially in an amount of 90-97wt.%, based on the total weight of the pharmaceutical composition, and one or more extragranular excipients, the total weight of extragranular excipients being in an amount of 1-70wt%, preferably in an amount of 2-30wt.%, more preferably in an amount of 3-15wt.%, especially in an amount of 3-10wt.%, based on the total weight of the pharmaceutical composition. According to one embodiment, the pharmaceutical composition of the present invention is a coated comprimate comprising a comprimate and a coating coated on the comprimate, (e.g. a coated tablet comprising a tablet core and a coating coated on the tablet core), wherein the comprimate comprises: one or more apixaban-containing granules, said one or more apixaban-containing granules being granules comprising hydroxyethyl cellulose (especially as disclosed herein), in an amount of 30-99wt.%, preferably in an amount of 70-98wt%, more preferably in an amount of 85-97wt.%, especially in an amount of 90-95wt.%, based on the total weight of the pharmaceutical composition, and
one or more extragranular excipients, the total weight of extragranular excipients being in an amount of 1-70wt%, preferably in an amount of 2-30wt.%, more preferably in an amount of 3-15wt.%, especially in an amount of 5-10wt.%, based on the total weight of the pharmaceutical composition.

In particular, the composition of the present invention can be prepared by technological procedures comprising wet granulation or melt granulation.

Wet granulation (especially for preparing HEC containing granules) can be performed with a granulation fluid comprising water. Wet granulation can be performed with a granulation fluid in the absence of organic solvents, especially in the absence of alcohols. Granulation fluid (used for wet granulation) preferably comprises water, and can be optionally free of organic solvent or can comprise not more than 10wt.%, preferably not more than 5 wt.% of organic solvent, based on the total weight of the granulation fluid. Especially, granulation fluid (used for wet granulation) can be optionally free of organic solvent or can comprise not more than 10 wt.%, preferably not more than 5 wt.% of organic solvent, based on the total weight of the granulation fluid. In the context of the present application, an organic solvent can be in particular a compound comprising a carbon atom, which compound is liquid at a temperature of 20°C, especially at a pressure of 101 325 Pa.

In particular, granulation fluid (especially for preparing HEC containing granules) comprising water can be free of one or more selected from the group of R^{a}-OH, R^{b}-O-R^{c}, R^{d}-C(O)-O-R^{e}, hydrocarbons, and mixtures thereof, wherein R^{a}, R^{b}, R^{c}, R^{d}, R^{e}, can be each independently selected from alkyls, e.g. C1-C10 alkyls, (such as e.g. methanol, ethanol, diethyl ether, ethyl acetate, etc.), dimethylsulfoxide, dimethylformamide, or can comprise not more than 10 wt.%, preferably not more than 5 wt.% of said one or more selected from the group of R^{a}-OH, R^{b}-O-R^{c}, R^{d}-C(O)-O-R^{e}, hydrocarbons, dimethylsulfoxide, dimethylformamide, and mixtures thereof, based on the total weight of the granulation fluid. In particular, granulation fluid can consist of water or can comprise at least 60 wt.-%, optionally at least 70 wt.%, further optionally at least 75 wt.% of water, based on the total weight of the granulation fluid.

Wet granulation with granulation fluid can comprise contacting the mixture or the compound to be granulated with granulation fluid, e.g. said contacting can be or comprise spraying of granulation fluid on the mixture or compound to be granulated.

One or more excipients selected from pH modifiers, diluents, binders, disintegrants, lubricants, glidants, antitacking agents can optionally be dissolved, suspended, emulsified or dispersed into the granulation fluid which is sprayed onto the mixture, in particular powder mixture, comprising apixaban and at least one excipient or a mixture of excipients.

In an embodiment of the present invention, granulation can be performed using granulator(s) (especially state of the art granulator(s)) such as high shear granulator, low shear granulator, or a fluid bed granulator.

The mixing of apixaban and at least one excipient or a mixture of excipients may be effected in conventional devices used for mixing of powder or powders, e.g. motionless (passive) mixers, fluidized bed, diffusion, biconic diffusion, uni- or biconic, tubular, cubic, planetary, Y-, V-shaped or high-shear mixers. The same equipment may be used in the preparation of compression mixture with the prior step of a granulate preparation by a wet granulation.

For drying of the granulate, prepared by wet granulation, conventional drying devices such as a fluid-bed dryer or drying chambers can be used.

Conventional equipment can be used for applying a film coating, such as a Wurster coating system or conventional perforated or non-perforated coating pans for use in pharmaceutical industry.

Any possible combination of incorporation of apixaban and polymer can be used in the wet granulation process.

It is particularly preferred that the composition according to the present invention comprises granulate (i.e. intragranular phase) and an extragranular phase. Intra- and extragranular phase together comprise the compression mixture. The compression mixture can comprise or can consist of intragranular and extragranular phase.

In one embodiment, it is preferred that granulate (which can be e.g. HEC containing granulate or polymer component containing granulate) can be prepared by wet granulation. It is particularly preferred that the wet granulation is performed in fluid-bed granulator/fluid-bed dryer.

A pharmaceutical composition comprising or consisting of one or more apixaban-containing granules, especially a pharmaceutical composition consisting of one or more apixaban-containing granules, can be filled into capsule(s) or sachet(s) or can be compressed in one or more comprimate(s), preferably in one or more tablet(s) or microtablet(s), or may be formulated in one or more pellet(s), which comprimate(s) or pellet(s) optionally can be coated and/or optionally filled into capsule(s) or sachet(s).

In another aspect, there is provided a pharmaceutical composition comprising apixaban, said pharmaceutical composition comprising one or more apixaban-containing granules, wherein each of said apixaban-containing granules comprises or consists of (i) apixaban, and (ii) hydroxyethyl cellulose, which is obtainable by a process for preparing a pharmaceutical composition disclosed herein, especially by a process as defined in the claims annexed.

The terms "melt extrudate" and "extrudate prepared by melt extrusion" and the terms "hot melt extrudate" and "extrudate prepared by hot melt extrusion" can be used interchangeably herein.

Melt extrusion can be in particular hot melt extrusion (HME). Melt extrusion, especially HME, are procedures known to the skilled person. Hot Melt extrusion comprises moving of material at a temperature of preferably at least 110°C, especially at least 190°C, further especially at least 210°C, through a nozzle. Melt extrusion (in particular HME) can be carried out in an extruder which can comprise a filling device and a shaft ensuring movement of the material to be extruded through a heated section and an extruding device (e.g. an extruding head). The time period that the material to be extruded remains in the extruder can be e.g. less than 9 minutes, especially 6 to 8 minutes. Especially, HME can comprise pumping to the mixture to be subjected to HME at elevated temperature, especially of at least 190°C, further especially at least 210°C, through a heated barrel to obtain an extrudate. An extruder can consist of one or two rotating screws inside a (usually cylindrical) barrel.

The granules may be granules of any shape, especially particles of any shape, including but not limited to the shape of granules obtainable by wet granulation, dry granulation, melt granulation, especially hot-melt extrusion, optionally in combination with milling and/or sieving procedures.

The one or more apixaban-containing granules can be in particular apixaban-containing extrudate obtained in step (B) or sieved and/or milled apixaban-containing extrudate obtained in step (C) of the process for the preparation of an apixaban-containing extrudate, described herein. The term milling as used herein comprises any procedure resulting in a reduction of the size of the starting material subjected to milling. In an embodiment, milling can be carried out in one or more milling devices; milling devices, especially milling devices which may be used in the pharmaceutical field are known to the skilled person.

The term "pharmaceutical composition comprising" encompasses among others the meaning "pharmaceutical composition consisting of".

The following examples illustrate the invention and are not intended to restrict the scope of the invention in any way.

### Examples

### Example 1 and Reference Examples 1 to 4

### Process for the preparation of the pharmaceutical compositions according to Example 1 and Reference Examples 1 to 4

The size of laboratory experiments was 5.000 tablets.

Apixaban was mixed with hydroxyethyl cellulose (or other binder as per reference examples 1-4 according to table 1), sodium lauryl sulfate and part of lactose. The mixture was sieved through a mesh with screen size 30#. Triturate of apixaban was then mixed with microcrystalline celullose and the rest of lactose.

Granulating mixture was put in a 10 L high-shear mixer where it was first dry blended for 2 min. Water was then sprayed on the mixture at the rate between 40 - 200 g/min until sufficient agglomeration was obtained. After homogenization of the granulate for 1 min, the granulate was dried in a fluid-bed at inlet air temperature of at least 30 °C until less than 3 % loss on drying (105 °C, 5 min) was achieved. The dried granulate was then sieved through a mesh with screen size 18#.

Tableting mixture was prepared by first mixing the granulate with sodium croscarmellose and then magnesium stearate was added.

Tableting was performed on a rotary tablet press. Tablet cores were then coated using standard coating procedure until the desired mass gain was achieved.

Table 1 summarizes the ingredients used for preparing the pharmaceutical compositions according to Example 1 (comprising hydroxyethyl cellulose as a binder) and according to References Examples 1 to 4, respectively.

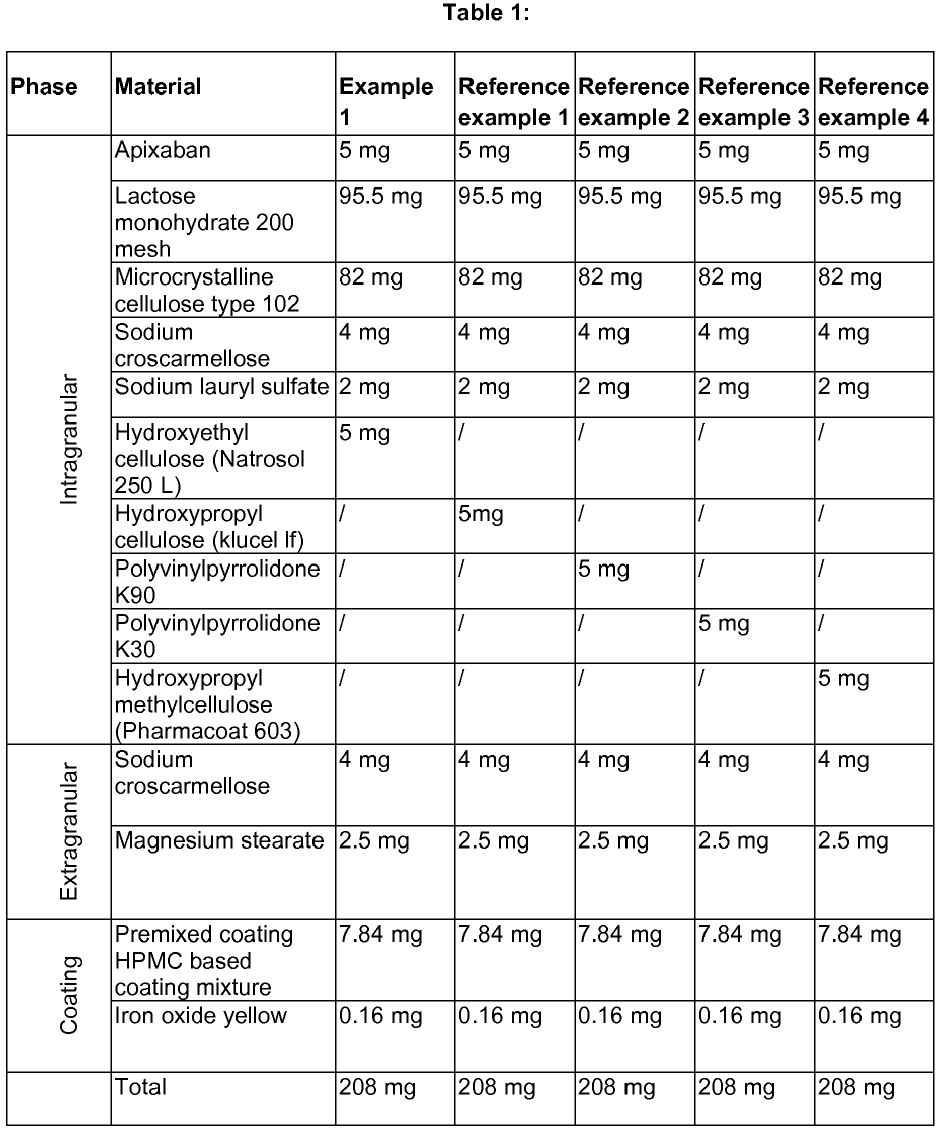

### Examples 2 to 9

Examples 2 to 9 below are prepared in the same manner as described for Example 1 supra. Examples 2 to 9 show that the advantageous properties of the pharmaceutical composition remain essentially unchanged, even when hydroxyethyl cellulose in the form of different commercially available products (especially hydroxyethyl cellulose having different viscosity grades) are used.

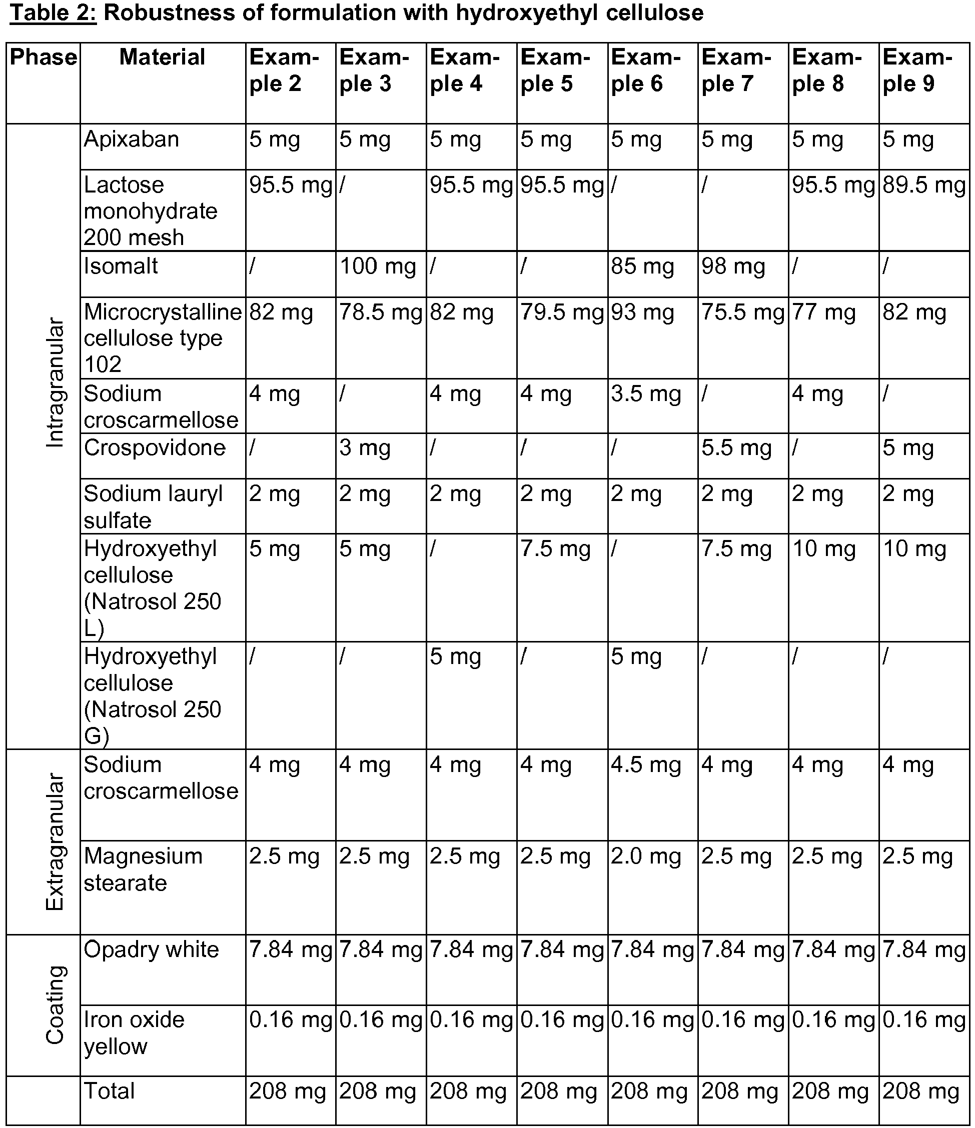

### Example 10: Dissolution rates for a composition according to Example 1 and for compositions according to reference examples 1 to 4

Table 3 below shows the dissolution rates for a composition according to Example 1 and the Reference Examples according to Table 1.

**Table 3**

| | Dissolved apixaban [%] | | | | |
|---|---|---|---|---|---|
| Time [min] | Example 1 | Reference example 1 | Reference example 2 | Reference example 3 | Reference example 4 |
| 0 | 0 | 0 | 0 | 0 | 0 |
| 5 | 47 | 17 | 15 | 27 | 13 |
| 10 | 62 | 42 | 37 | 49 | 37 |
| 15 | 70 | 57 | 48 | 57 | 53 |
| 20 | 75 | 64 | 56 | 64 | 60 |
| 30 | 82 | 72 | 65 | 72 | 69 |

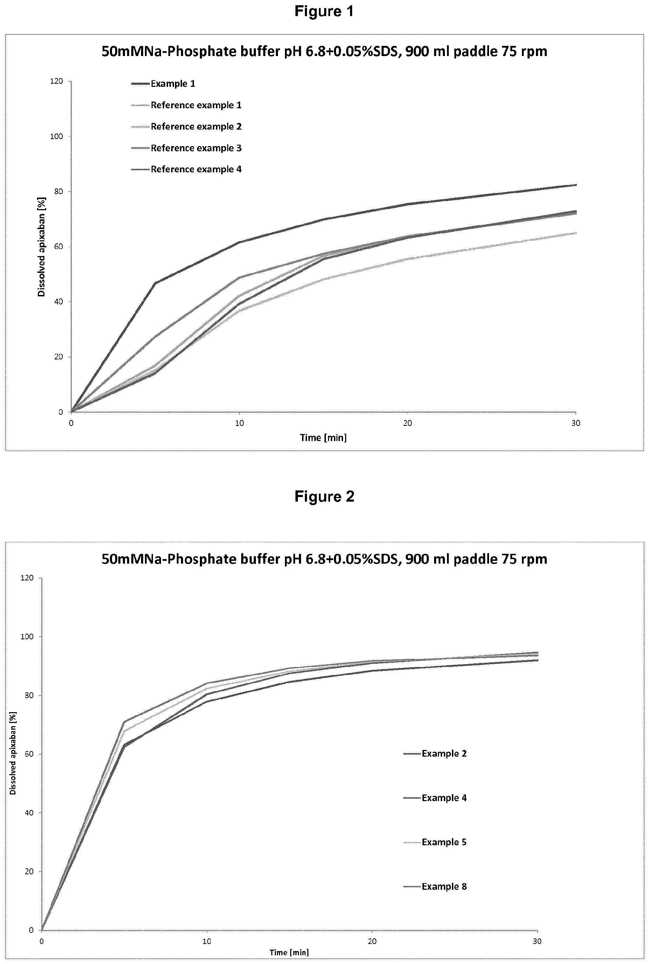

Figure 1 annexed illustrates dissolution rates of Example 1 and Reference Examples 1-4, respectively, and shows the improved dissolution properties of the present invention.

Table 4 below shows dissolution rates for Examples 2, 4, 5 and 8 (with composition as shown in Table 2), and supports robustness of dissolution even when different amounts and viscosity grades of binder are applied.

**Table 4: Dissolution rates for Examples 2, 4, 5 and 8 (according to table 2) showing robustness of dissolution regarding different amounts and viscosity grades of binder**

| | Dissolved apixaban [%] | | | |
|---|---|---|---|---|
| **Time [min]** | **Example 2** | **Example 4** | **Example 5** | **Example 8** |
| 0 | 0 | 0 | 0 | 0 |
| 5 | 63 | 62 | 68 | 71 |
| 10 | 78 | 80 | 82 | 84 |
| 15 | 85 | 87 | 88 | 89 |
| 20 | 88 | 91 | 91 | 92 |
| 30 | 92 | 94 | 94 | 94 |

Figure 2 annexed illustrates the dissolution rates of Examples 2, 4, 5 and 8, which show a consistent dissolution of for the examples according to the present invention irrespective of variations in quantity and quality of the binder.

The dissolution testing illustrated in Figures 1 and 2 annexed was carried out in 900 ml of dissolution medium (50 mM Natrium-Phosphate buffer having pH 6.8 and containing 0.05 wt.-% SDS (sodium dodecyl sulfate)). The dissolution testing was performed during 30 minutes at a rotation speed of 75 rpm. The dissolution testing is carried out at a temperature of the dissolution medium of 37°C±0.5°C (especially 37°C). The apparatus used for dissolution testing was USP apparatus II (Paddle).

### Example 11: Process for preparation of hot-melt extrudate

The size of laboratory experiments was 0,5 kg of extrudate.

Apixaban was mixed with polymer at the investigated ratio in a suitable mixer. Twin screw extruder was used to extrude the mixture at the desired temperature and feed rate. Standard configuration of the screws was used and the speed was set to 70 - 110 rpm. Hot-melt extrudate was extruded through a die with 2 mm in diameter, cooled on a cooling belt and grinded to smaller size. Grinded extrudate was then sieved and subjected to stability testing under controlled conditions of 40-50 °C and 75 % RH.

**Table 5**

| **Examples** | **Apixaban: Copovidone** | Maximum set T of extrusion [°C] | Form of apixaban in the extrudate | Stability testing |
|---|---|---|---|---|
| Example 11-1 | 1:4 | 190 | not determined | / |
| Example 11-2 | 1:6 | 190 | not determined | / |
| Example 11-3 | 1:6 | 200 | Pure amorph. form | not determined |
| Example 11-4 | 1:6 | 210 | Pure amorph. form | 37d / 50°C-75 % RH / Pure amorph. form |
| Example 11-5 | 1:9 | 190 | Pure amorph. form | 1 m/ 40°C-75% RH / Pure amorph. form |
| Example 11-6 | 1:9 | 210 | Pure amorph. form | 1m/40°C-75% RH/ Pure amorph. form |
| **Example** | **Apixaban: PCA-PVA-PEG (Soluplus®)** | Maximum set T of extrusion [°C] | Form of apixaban in the extrudate | Stability testing |
| Example 11-7 | 1:6 | 230 | Pure amorph. form | / not determined |
| **Example** | **Apixaban: Parteck MXP** | Maximum set T of extrusion [°C] | Form of apixaban in the extrudate | Stability testing |
| Example 11-8 | 1:6 | 210 | Pure amorph. form | not determined |

| | | | | |
|---|---|---|---|---|
| d...day, m...month | | | | |

### Examples 12 - 17: Process for the preparation of the pharmaceutical compositions according to Examples 12 -17

The size of laboratory experiments was 5.000 tablets.

Tableting mixture was prepared by first mixing the sieved extrudate (prepared as per example 7) with sodium lauryl sulfate, lactose anhydrous, microcrystalline cellulose type 102 and sodium croscarmellose and then magnesium stearate was added.

Tableting was performed on a rotary tablet press. Tablet cores were then coated using standard coating procedure until the desired mass gain was achieved.

Table 6 summarizes the ingredients used for preparing the pharmaceutical compositions according to Example 12 - 17.

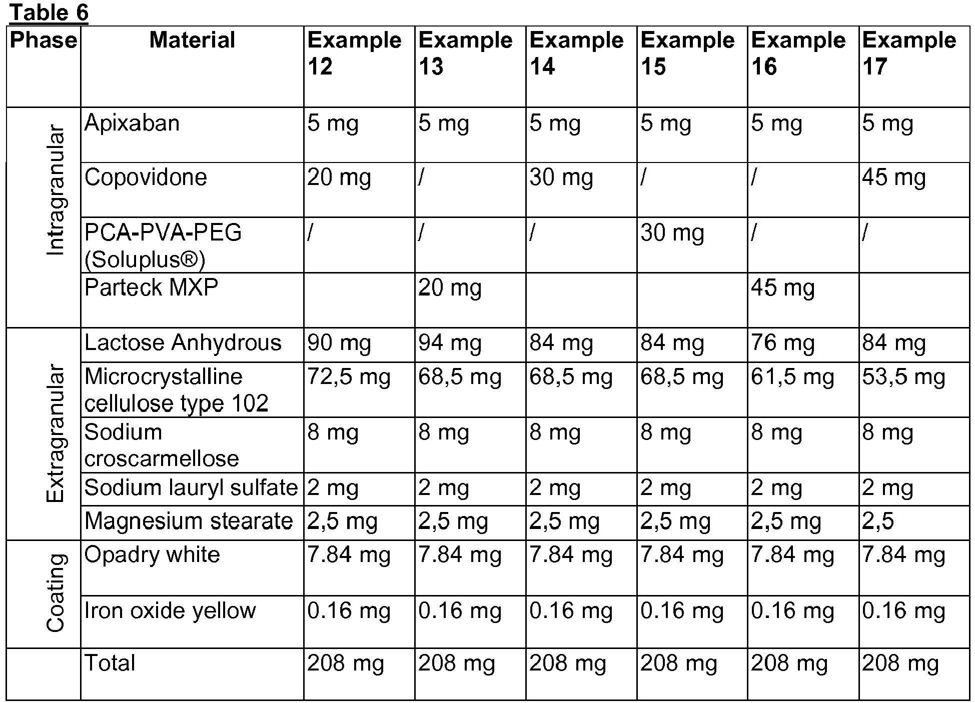

### Examples 18 - 19: Process for the preparation of the pharmaceutical compositions according to Examples 18-19

The size of laboratory experiments was 5.000 tablets.

Granulating liquid was prepared by dissolving hydroxypropylcellulose in sufficient amount of ethanol 96 %.

Granulating mixture was prepared by mixing the sieved extrudate (prepared as per example 7) with lactose monohydrate, microcrystalline cellulose type 101 and sodium croscarmellose. Granulating mixture was then put in a 10 L high-shear mixer where it was first dry blended for 2 min. Granulating liquid was then sprayed on the mixture until sufficient agglomeration was obtained. After kneading of the granulate for 1 min, the granulate was dried in a fluid-bed at inlet air temperature of at least 30 °C until less than 3 % loss on drying (105 °C, 5 min) was achieved. The dried granulate was then sieved through a mesh with screen size 18#.

Tableting mixture was prepared by first mixing the granulate with lactose anhydrous, sodium croscarmellose, microcrystalline cellulose type 102, sodium lauryl sulfate and then magnesium stearate was added.

Tableting was performed on a rotary tablet press. Tablet cores were then coated using standard coating procedure until the desired mass gain was achieved.

Table 7 summarizes the ingredients used for preparing the pharmaceutical compositions according to Example 18 and 19, respectively.

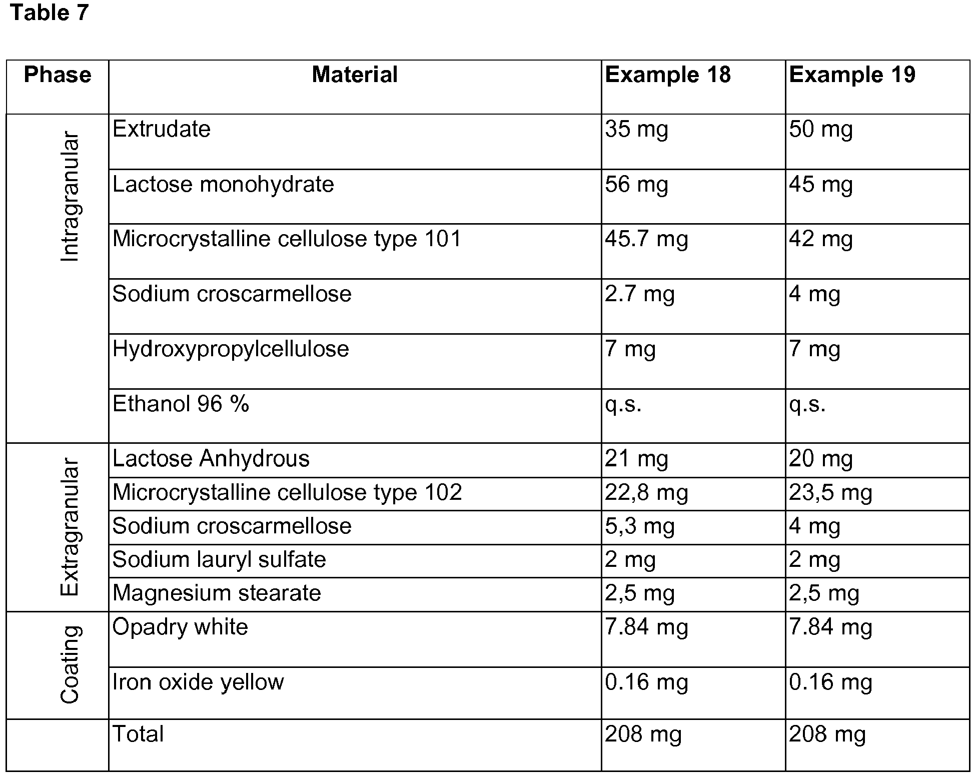

### Examples 20 - 21: Process for the preparation of the pharmaceutical compositions according to Examples 20-21

The size of laboratory experiments was 5.000 tablets.

Triturate was prepared by first mixing the sieved extrudate (prepared as per example 7) with sodium lauryl sulfate and microcrystalline cellulose type 101. Then mannitol, polyethylene glycol and macrogol were added and the mixture was sieved through a mesh with screen size 0.6 mm.

Granulating mixture was then put in a 10 L high-shear mixer with temperature regulation. During the mixing, the heating was applied until sufficient agglomeration was obtained. The granulate was cooled on a tray and sieved through a mesh with screen size 21C.

Tableting mixture was prepared by first mixing the granulate with sodium croscarmellose and colloidal silicon dioxide and then magnesium stearate was added.

Tableting was performed on a rotary tablet press. Tablet cores were then coated using standard coating procedure until the desired mass gain was achieved.

Table 8 summarizes the ingredients used for preparing the pharmaceutical compositions according to Example 20 and 21, respectively.

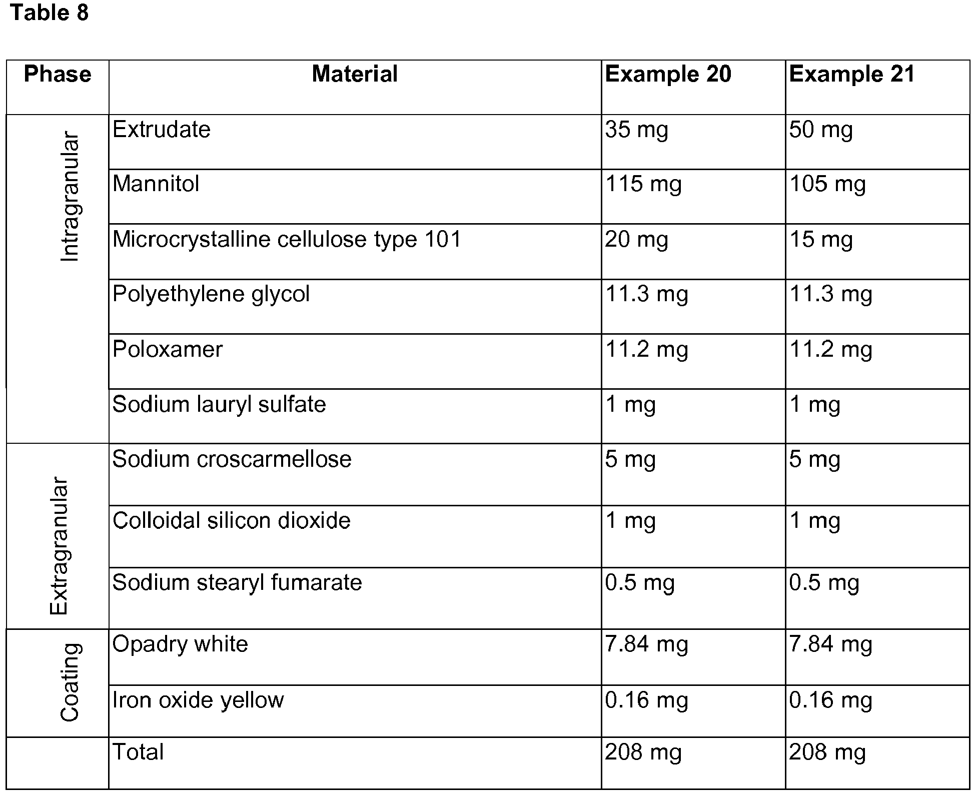

### Examples 22 - 23: Process for the preparation of the pharmaceutical compositions according to Examples 22-23

The size of laboratory experiments was 5.000 tablets.

Compacting mixture was prepared by mixing the sieved extrudate (prepared as per example 7) with lactose monohydrate, microcrystalline cellulose type 802, sodium croscarmellose and sodium lauryl sulfate and then magnesium stearate was added.

Compacting mixture was compressed on roller compactor with the pressure sufficient to obtain ribbons with appropriate density. Ribbons were then milled through a mesh with screen size 18#.

Tableting mixture was prepared by first mixing the granulate with sodium croscarmellose and then magnesium stearate was added.

Tableting was performed on a rotary tablet press. Tablet cores were then coated using standard coating procedure until the desired mass gain was achieved.

Table 9 summarizes the ingredients used for preparing the pharmaceutical compositions according to Example 22 and 23, respectively.

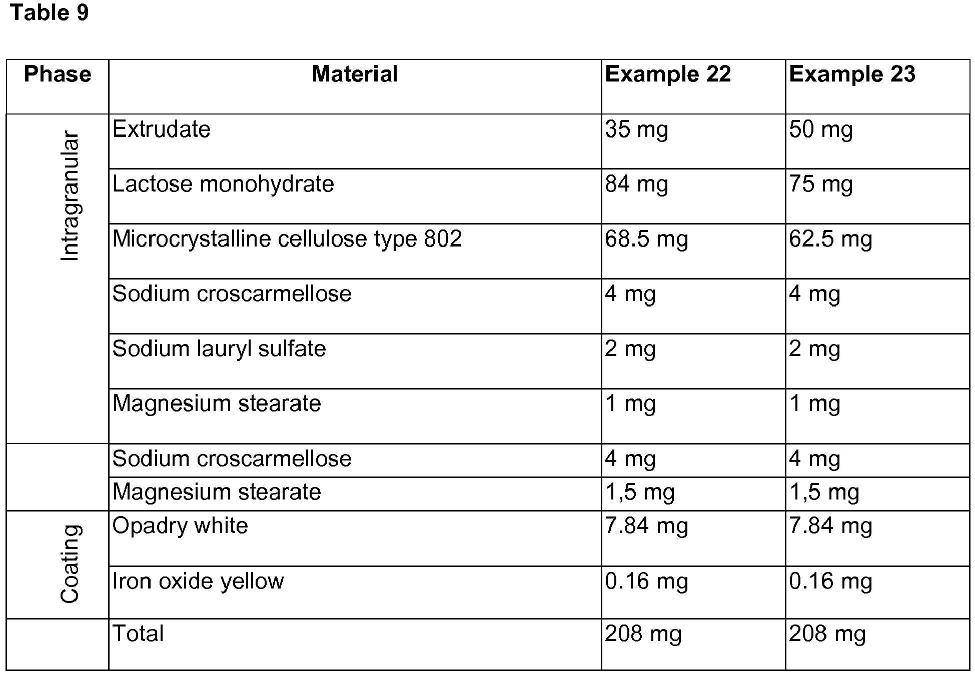

## Claims

1. Pharmaceutical composition comprising apixaban,
said pharmaceutical composition comprising or consisting of one or more apixaban-containing granules,
wherein each of said apixaban-containing granules comprises or consists of
(i) apixaban, and
(ii) hydroxyethyl cellulose.

2. Pharmaceutical composition according to claim 1, wherein each of said apixaban-containing granules has a weight ratio of apixaban : hydroxyethyl cellulose which is in the range from 1:10 to 10:1, preferably in the range from 1:5 to 5:1, more preferably in the range from 1:2 to 2:1, especially in the range from 1:1.6 to 1.6:1.

3. Pharmaceutical composition according to any one of the preceding claims, wherein each of said apixaban-containing granules is prepared by wet granulation in the presence of a granulation fluid comprising or consisting of water.

4. Pharmaceutical composition according to any one of the preceding claims, wherein each of said apixaban-containing granules comprises or consists of
a) apixaban, hydroxyethyl cellulose, and a diluent, or
b) apixaban, hydroxyethyl cellulose, and a disintegrant, or
c) apixaban, hydroxyethyl cellulose, and a surfactant, or
d) apixaban, hydroxyethyl cellulose, a diluent, and a disintegrant, or
e) apixaban, hydroxyethyl cellulose, a diluent, and a surfactant, or
f) apixaban, hydroxyethyl cellulose, a diluent, a disintegrant and a surfactant.

5. Pharmaceutical composition according to any one of the preceding claims, wherein each of said apixaban-containing granules comprises or consists of apixaban, hydroxyethyl cellulose, and a diluent and has a weight ratio of apixaban : diluent which is in the range from 1:10 to 1:60, preferably from 1:20 to 1:50, more preferably from 1:30 to 1:40, especially from 1:34 to 1:38, or
wherein the diluent is or comprises diluent selected from the group consisting of carbohydrates and derivatives thereof, sugar alcohols, celluloses, starch and derivatives thereof, magnesium aluminometasilicate, calcium salts of phosphoric acid, alkali carbonates, earth alkali carbonates, alkali hydrogencarbonates, earth alkali hydrogencarbonates, and mixtures thereof,
optionally the diluent is or comprises diluent selected from the group consisting of lactose, cellulose, and mixtures thereof,
optionally wherein the diluent is a mixture comprising or consisting of lactose monohydrate and microcrystalline cellulose.

6. Pharmaceutical composition comprising apixaban,
said pharmaceutical composition comprising or consisting of apixaban-containing extrudate, in particular apixaban-containing hot-melt extrudate,
wherein said apixaban-containing extrudate, in particular apixaban-containing hot-melt extrudate, comprises or consists of
(I.) apixaban, and
(II.) a polymer component which is or comprises polymer selected from polyvinyl caprolactam-polyvinyl acetate-polyethylene glycol graft copolymer, copovidone, poly(vinyl alcohol), and mixtures thereof, and
wherein said apixaban-containing extrudate, in particular apixaban-containing hot-melt extrudate, has a weight ratio between apixaban and polymer component (apixaban : polymer component), which weight ratio is 1:4 or less, especially is 1:6 or less, especially is in the range from 1:6 to 1:9,
wherein optionally said apixaban-containing extrudate, in particular apixaban-containing hot-melt extrudate, is obtainable from a process comprising extrusion, in particular hot melt extrusion, carried out at a temperature of at least 190°C, especially at a temperature in the range from 190°C to 230°C.

7. Pharmaceutical composition comprising apixaban,
said pharmaceutical composition comprising or consisting of one or more apixaban-containing granules,
wherein each of said apixaban-containing granules comprises or consists of
(α) apixaban-containing extrudate, in particular apixaban-containing hot-melt extrudate, comprising or consisting of
(I.) apixaban, and
(II.) a polymer component which is or comprises polymer selected from polyvinyl caprolactam-polyvinyl acetate-polyethylene glycol graft copolymer, copovidone, poly(vinyl alcohol), and mixtures thereof, wherein optionally said apixaban-containing extrudate, in particular apixaban-containing hot-melt extrudate, optionally has a weight ratio between apixaban and polymer component (apixaban : polymer component), which weight ratio is 1:4 or less, especially is 1:6 or less, especially is in the range from 1:6 to 1:9, and
(β) optionally one or more intragranular excipients.

8. Pharmaceutical composition according to any one of the preceding claims, wherein the pharmaceutical composition is:
(a.) a pharmaceutical composition comprising:
(A) one or more apixaban-containing granules, wherein each of said apixaban-containing granules comprises or consists of
(i) apixaban, and
(ii) hydroxyethyl cellulose, and
(B) one or more extragranular excipients, wherein preferably said one or more extragranular excipients comprise a disintegrant,
or wherein the pharmaceutical composition is:
(b.) a pharmaceutical composition comprising:
(A') one or more apixaban-containing granules, wherein each of said apixaban-containing granules comprises or consists of
(α)apixaban-containing extrudate, in particular apixaban-containing hot-melt extrudate, comprising or consisting of
(I.) apixaban, and
(II.) a polymer component which is or comprises polymer selected from polyvinyl caprolactam-polyvinyl acetate-polyethylene glycol graft copolymer, copovidone, poly(vinyl alcohol), and mixtures thereof, and
(β) optionally one or more intragranular excipients, and
(B) one or more extragranular excipients, wherein preferably said one or more extragranular excipients comprise a disintegrant.

9. Pharmaceutical composition according to any one of the preceding claims, wherein said pharmaceutical composition is a comprimate, especially a tablet.

10. Pharmaceutical composition according to any one of claims 1-5, 8-9, wherein each of said apixaban-containing granules comprise:
apixaban in an amount of 0.7-30wt.%, preferably 1-15wt.%, more preferably 1.5-9wt.%, even more preferably 2-5wt.%, based on the total weight of the one or more apixaban-containing granules,
hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
optionally diluent in an amount of 50-99wt.%, preferably 70-97wt.%, more preferably 85-95wt.%, based on the total weight of the one or more apixaban-containing granules,
optionally disintegrant in an amount of 0.5-10wt.%, preferably 1-8wt.%, more preferably 1.5-6wt.%, even more preferably 2-4wt.%, based on the total weight of the one or more apixaban-containing granules,
optionally surfactant in an amount of 0.1-5wt.%, preferably 0.5-2wt.%, more preferably 0.7-1.8wt.%, based on the total weight of the one or more apixaban-containing granules, and
optionally one or more further excipients.

11. Pharmaceutical composition according to any one of claims 1-5, 8-10, wherein each of said apixaban-containing granules comprises or consists of:
- apixaban in an amount of 0.7-30wt.%, preferably 1-15wt.%, more preferably 1.5-9wt.%, even more preferably 2-5wt.%, based on the total weight of the one or more apixaban-containing granules,
- hydroxyethyl cellulose in an amount of 0.5-30wt.%, preferably 1-15wt.%, more preferably 1.5-9wt.%, even more preferably 2-5wt.%, based on the total weight of the one or more apixaban-containing granules,
- lactose, in particular lactose monohydrate, in an amount of 25-60wt.%, preferably 38-55wt.%, more preferably 44-51wt.%, based on the total weight of the one or more apixaban-containing granules,
- microcrystalline cellulose in an amount of 25-55wt.%, preferably 38-49wt.%, more preferably 40-48wt.%, based on the total weight of the one or more apixaban-containing granules,
- sodium croscarmellose in an amount of 0.5-10wt.%, preferably 1-8wt.%, more preferably 1.5-6wt.%, even more preferably 2-4wt.%, based on the total weight of the one or more apixaban-containing granules,
- sodium lauryl sulfate in an amount of 0.1-5wt.%, preferably 0.5-2wt.%, more preferably 0.7-1.8wt.%, based on the total weight of the one or more apixaban-containing granules, and
- optionally one or more further excipients.

12. Pharmaceutical composition according to any one of claims 8-11, wherein the pharmaceutical composition is a pharmaceutical composition comprising:
(A) one or more apixaban-containing granules, wherein each of said apixaban-containing granules comprises or consists of
(i) apixaban, and
(ii) hydroxyethyl cellulose, and
(B) one or more extragranular excipients, wherein preferably said one or more extragranular excipients comprise a disintegrant, and
wherein the pharmaceutical composition comprises:
one or more apixaban-containing granules in an amount of 30-99wt.%, preferably in an amount of 70-98wt%, more preferably in an amount of 85-97wt.%, especially in an amount of 90-95wt.%, based on the total weight of the pharmaceutical composition, and
one or more extragranular excipients, the total weight of extragranular excipients being in an amount of 1-70wt%, preferably in an amount of 2-30wt.%, more preferably in an amount of 3-15wt.%, especially in an amount of 5-10wt.%, based on the total weight of the pharmaceutical composition.

13. Process for preparing a pharmaceutical composition comprising apixaban, in particular pharmaceutical composition for oral administration, said process comprising
(i) preparing a granulate comprising apixaban and hydroxyethyl cellulose, preferably by wet granulation;
(ii) optionally drying the granulate obtained from step (i),
(iii) optionally sieving and/or milling the granulate obtained from step (i) or the dried granules obtained from step (ii),
(iv) optionally combining the granulate obtained from step (i) or the optionally dried granules obtained from step (ii) or the optionally sieved and/or milled granules obtained from optional step (iii) with at least one excipient to obtain a compression mixture;
(v) optionally compressing the compression mixture to obtain a comprimate, in particular tablet; and
(vi) optionally applying a coating on the comprimate.

14. Process for the preparation of an apixaban-containing extrudate, and optionally for the preparation of a granulate or a comprimate, said apixaban-containing extrudate comprising or consisting of (I) apixaban, and (II) a polymer component which is or comprises polymer selected from polyvinyl caprolactam-polyvinyl acetate-polyethylene glycol graft copolymer, copovidone, poly(vinyl alcohol), and mixtures thereof,
which process comprises:
(A) preparing a mixture comprising (I) apixaban, and (II) a polymer component which is or comprises polymer selected from polyvinyl caprolactam-polyvinyl acetate-polyethylene glycol graft copolymer, copovidone, poly(vinyl alcohol), and mixtures thereof, optionally apixaban and said polymer component being present in said mixture in a weight ratio apixaban :
polymer component which is 1:4 or less, especially is 1:6 or less, especially is in the range from 1:6 to 1:9;
(B) subjecting the mixture obtained from step (A) to extrusion, especially hot melt extrusion, optionally the hot melt extrusion being carried out at a temperature of at least 190°C, further optionally at a temperature in the range of from 190°C to 230°C;
(C) optionally comminuting the apixaban-containing extrudate obtained in step (B) to obtain comminuted apixaban-containing extrudate;
(D) optionally converting the apixaban-containing extrudate obtained in step (B) or the comminuted apixaban-containing extrudate obtained in step (C) to form a granulate;
(E) optionally mixing the apixaban-containing extrudate obtained in step (B) or the comminuted apixaban-containing extrudate obtained in step (C) or the granulate obtained in step (D) with at least one excipient to obtain a compression mixture;
(F) optionally compressing the compression mixture to obtain a comprimate, in particular tablet; and
(G) optionally applying a coating on the comprimate.

15. Pharmaceutical composition according to any one of claims 1 to 12 for use in treating or preventing a thromboembolic disorder or disease, in particular a venous thromboembolic disorder or disease.
